# EUROPEAN PATENT APPLICATION

(11) **EP 4 467 155 A1**
(43) Date of publication of application: **27.11.2024**
(21) Application number: 23382478.8
(22) Date of filing: 23.05.2023
(51) Int. Cl.: A61K 39/00, A61P 37/00, C07K 14/725, C07K 14/705, C12N 15/62

(54) **CAR-THYTREG CELLS, COMPOSITIONS AND USES THEREOF IN IMMUNOTHERAPY**

(71) Applicant: Fundación para la Investigación Biomédica del Hospital Gregorio Marañón (FIBHGM), 28007 Madrid (ES)
(72) Inventor: PION, Marjorie, 28007 Madrid (ES); CORREA ROCHA, Rafael, 28007 Madrid (ES); GALLEGO VALLE, Jorge, 28007 Madrid (ES)
(74) Representative: Hoffmann Eitle

(57) **Abstract**

The invention provides a thymus T regulation cell (ThyTreg cell) which codes for, or alternatively expresses on its surface, a chimeric antigen receptor (CAR) comprising an extracellular domain, a hinge region, a transmembrane domain and an intracellular domain, wherein the intracellular domain comprises: a cytoplasmatic co-stimulation domain with a sequence having an identity of at least 85% with respect to SEQ ID NO: 1; and a cytoplasmatic stimulation domain.

The invention also provides compositions and uses in immunotherapy.

## Description

### FIELD OF THE INVENTION

The present invention falls within clinical immunology and cell-advanced immunotherapy. In particular, the present invention provides CAR-ThyTreg cells, compositions comprising thereof and uses thereof in inducing a specific immune tolerance.

### BACKGROUND

The immune system's main function is to defend the organism from pathogenic agents, and it's also in charge of eliminating tumour cells and preventing cancer development. However, it can also give rise to inadequate responses through the appearance of autoimmune processes, allergies, or transplant rejections. The proper functioning of the immune system is only possible if there is a balance or homeostasis adequate thereto. An excessive response will give rise to pathologies such as allergies or rejection, and a defective response will enable the progression of infections and cancer.

Autoimmune diseases, hyper-inflammatory processes, and immune rejection are currently treated with immunosuppressive drugs. Despite improvements, they still do not offer a definitive solution to these diseases and continue to cause side effects affecting the patient's clinical evolution. Specifically, long-term immunosuppression causes chronic toxicity, which, in addition to significantly influencing the patient's quality of life, affects the fulfilment of the treatment. Since most immunosuppressors act in a non-selective way, the entire system is repressed and/or deregulated, which can interfere with normal immune system function, which may have lifelong consequences.

To this end, achieving immune tolerance that will indefinitely avoid graft rejection or autoimmune disease symptoms that affect millions of people has become the major challenge of modern medicine. The current opinion of the science community is that just one immune tolerance induction that involves re-educating the recipient's immune response will allow the indefinite survival of the graft, the limitation of the autoimmune symptoms, or the restoration of homeostasis in hyperinflammation processes.

However, no therapies have been developed so far and found at the commercial level with the ability to induce immune tolerance, to prevent or cure autoimmune diseases, allogeneic graft rejection, and suppressing hyper-inflammation pathologies. One of the most promising alternatives is to induce immune tolerance through cell immunotherapy (Sicard et al., 2015).

The discovery of a subset of lymphocytes with a suppressive capacity capable of inducing this tolerance has generated widespread enthusiasm in the clinical sphere. These cells, called regulatory T cells (Treg), constitute an essential part of the immune system, and could play a crucial role in maintaining immune homeostasis beneficial to patients. Tregs can suppress the effector function of many cells, including T CD4+ and CD8+ lymphocytes, Natural Killer cells (NK cells), B cells, macrophages, and dendritic cells (DC) (Sakaguchi et al., 2008).

The primary role of Tregs in transplants has been confirmed by various studies in animal models of skin and heart transplants, demonstrating that the Tregs present in the receptacle at the time of the transplant are critical to the induction and maintenance of tolerance to the graft (Juneja et al., 2022). These Treg cells will impede the activation and expansion of effector T cells, which are responsible for cellular rejection. Tregs can also induce the death of B cells, preventing humoral rejection, as already demonstrated in a cardiac xenotransplantation model (Ma et al., 2008). Cell therapy with Tregs is therefore postulated to become the great hope in the treatment of diseases mediated by an excessive or inadequate response of the immune system, such as autoimmune processes (Bluestone et al., 2015), graft-versus-host disease in bone marrow transplant patients (Brunstein et al., 2016), or transplant rejection (Safinia et al., 2016). It is assumed that the mechanism of this therapy is based on that a greater number of circulating functional Tregs will be capable of preventing the activation and proliferation of effector cells that trigger these diseases. Treg cell transfer would substantially increase their number in circulation and thus potentiate the recipient's intrinsic tolerance mechanisms in the receptacle to the transplanted organ or own tissues.

The safety and potential effectiveness of Treg therapy in humans are reflected in the first Phase-I/II trials already performed. Most clinical trials with Tregs have been performed within the context of bone marrow transplants in patients with haematological neoplasia, showing that the infusion of Tregs in these patients reduces or prevents graft-versus-host disease (GvHD) (Trzonkowski et al., 2009). Treg-based therapies were also tested with good results in type-1 diabetes children (Marek-Trzonkowska et al., 2014) with a partial response after 1-3 years of follow-up (Gliwiński et al., 2020) or in multiple sclerosis (Chwojnicki et al., 2021).

Previous data have shown that antigen-specific Treg cells are more effective than non-specific polyclonal Treg obtained by ex *vivo* expansion in reducing graft rejection (Sagoo et al., 2011). Up to now, antigen-specific Treg (Ag-specific) are obtained with the co-culture of Treg cells and allogeneic antigen-presenting cells (APCs) such as dendritic cells or B cells, making it possible to enrich alloreactive Treg cells *in vitro.* The main limitation of these strategies is the reduced number of Treg and APC that can be isolated. The CAR (chimeric antigen receptor) technology may be suitable for generating Ag-specific ThyTreg to overcome these limitations (Beheshti et al., 2022). A CAR is a synthetic construct mimicking T-cell receptor activation and redirects specificity and effector function toward a specified antigen when expressed in T cells. CARs comprise an extracellular region (scFv) responsible for binding to a particular antigen and an intracellular domain promoting T cell activity and proliferation.

Efforts have also been made in designing modified structures to enhance the innate properties of the Treg cells. In this regard, Levings M.K. and colleagues (Dawson et al., 2020) showed that the incorporation of the CD28 domain in the cytoplasmatic region of the CAR significantly enhanced the properties of the Treg cells, contrary to other constructs incorporating other fragments such as CD137 (also known as 41-BB).

Despite the efforts made, however, there remains still the need of further Treg-cell based therapeutic approaches suitable in the management of immune-related conditions.

### SUMMARY OF THE INVENTION

Prior to the present invention it was well-established the design of CAR-Treg cells based on the incorporation of CD28 domains in the cytoplasmatic region to potentiate their innate properties.

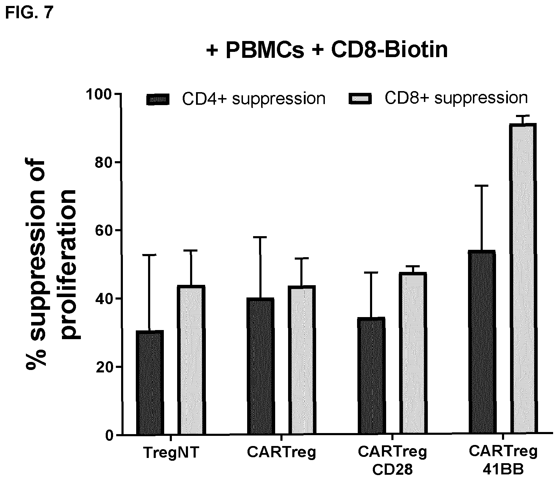

The inventors found, however, that in the case of thymus Treg cells (hereinafter also referred as ThyTreg cells), the incorporation of the CD28 domain within the cytoplasmatic domain of the CAR, provided a CAR-ThyTreg cell (hereinafter also referred as "CARCD28") with a low ability to suppress CD8+ cell populations. In FIG. 7, one can see that CARCD28 provided a CD8+-cell suppression effect of around 50%.

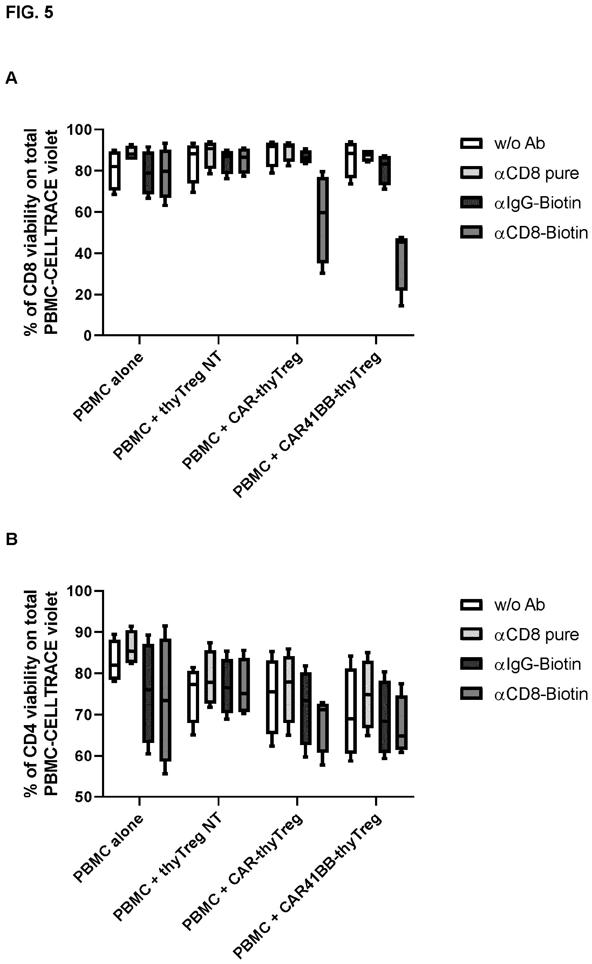

The present inventors have surprisingly found that when CAR-ThyTreg cells incorporated a 41-BB domain (SEQ ID NO: 1) within the cytoplasmatic region of the CAR (hereinafter also referred as CAR41 BB-ThyTreg cells), the ability to reduce CD8+ cell population was remarkably improved, reaching more than 90% of the population (FIG. 7). In addition, it was also found that not only the proliferation was down-regulated, but also the viability of CD8+-cells was also significantly reduced (FIG. 5). Therefore, the administration of the ThyTreg cells with this particular CAR construct, based on incorporating 41-BB sequence within the cytoplasmatic domain, provides a dual negative effect on the target CD8 population, acting at the level of viability, but also at the level of proliferation.

The inventors have also found that CAR41 BB-ThyTreg significantly affected the suppression of CD4 cell population (see FIG. 7).

These experimental data support the immunotolerance-enhancer effect provided by the incorporation of the 41-BB domain within CAR's cytoplasmatic domain on ThyTreg population.

The above is surprising in view of the prior art (Dawson et al., 2020) wherein it was reported that the incorporation of a 41-BB domain provided a reduced activity and that the significant activity was achieved when CD28 was included instead. Not only that, but also, this prior art did not provide any point at all to consider the incorporation of 4-1BB when designing a CAR-Treg cell with enhance immunotolerance.

Altogether, the data provided below show that the incorporation of 4-1 BB domain remarkably potentiates the innate properties of the ThyTreg cells, being suitable in the management of diseases and conditions induced or related to an over-activity of the immune system.

Therefore, in a first aspect the present invention provides a thymus T regulation cell (hereinafter referred as "ThyTreg cell") which codes for, or alternatively expresses on its surface, a chimeric antigen receptor (CAR) comprising an extracellular domain, a hinge region, a transmembrane domain, and an intracellular domain, wherein the intracellular domain comprises:
- a cytoplasmatic co-stimulation domain with a sequence having an identity of at least 85% with respect to SEQ ID NO: 1 (KRGRKKLLYIFKQPFMRPVQTTQEEDGCSCRFPEEEEGGCEL); and
- a cytoplasmatic stimulation domain.

Without being bound to the theory, the present inventors believe that the remarkably different behaviour of ThyTreg cells expressing a CAR including 41-BB, is due to the particular marker profile expressed on their surface, CD4+CD8+FOXP3.

In a second aspect, the present invention provides a process for preparing a CAR-ThyTreg cell population of the invention, the process comprising the step of transfecting a ThyTreg cell with an expression construct coding for the CAR as defined in the first aspect of the invention.

In a third aspect, the present invention provides the CAR-ThyTreg cell obtainable by the process of the second aspect of the invention.

In a fourth aspect, the present invention provides a pharmaceutical composition comprising a therapeutically effective amount of the ThyTreg cells object of the invention, together with one or more pharmaceutically acceptable excipients or carriers.

In a fifth aspect the present invention provides a combination comprising (a) a ThyTreg cell population as provided by the present invention functionalized with one member of a pair of specific binder moieties, particularly a member with the ability to bind to biotin, more particularly streptavidin, and (b) a binding moiety specific of an activated immune cell, which is conjugated to the second member of the pair of specific binder moieties, particularly conjugated to biotin or a fragment thereof.

In a sixth aspect, the present invention provides the CAR-ThyTreg cell as defined in the first or third aspect, or the combination of the fifth aspect of the invention, for use in therapy or diagnosis.

In a seventh aspect, the present invention provides the CAR-ThyTreg cell or the combination of the invention for use in inducing or restoring tolerance to the immune system. This aspect can be formulated as the use of a CAR-ThyTreg cell or the combination of the invention for the manufacture of a medicament in inducing or restoring tolerance to the immune system. This aspect can also be alternatively formulated as a method for inducing or restoring tolerance to the immune system in a subject, the method comprising administering an effective therapeutic amount of the CAR-ThyTreg cell of the invention, pharmaceutical composition, or combination, to a subject in need thereof.

Antigen-specific Tregs have been shown to be more effective in suppressing immune responses than polyclonal ones. Our study has shown that CAR-ThyTregs hold great promise due to their stable phenotypes and functions, and their ability to provide more potent and specific immunosuppression compared to polyclonal Tregs. Furthermore, we have found that the intracellular 41-BB signal is more effective in inducing suppressive function. This is important because 41-BB allows for less exhaustion of the cells, ensuring their durability in treated individuals and enabling them to effectively carry out their immunosuppressive function. This is particularly crucial in autoimmune diseases, immune rejection, allergies, and chronic inflammatory processes, where CAR-ThyTregs must combat chronic inflammatory states.

Thus, the present invention provides in a final aspect the CAR-ThyTreg cells, pharmaceutical composition, or combination of the invention for use in the treatment of a disease selected from: autoimmune disease, inflammatory processes, allergy, graft-versus host disease, or immune rejection to transplant. This aspect can be formulated as the use of CAR-ThyTreg cells, pharmaceutical compositions, or combinations of the invention for the manufacture of a medicament for the treatment of a disease selected from: autoimmune disease, inflammatory processes, allergy, graft-versus host disease, or immune rejection to transplant. This aspect can also be alternatively formulated as a method for the treatment of a disease selected from: autoimmune disease, inflammatory processes, allergy, graft-versus host disease, or immune rejection to transplant, the method comprising the step of administering a therapeutically effective amount of the CAR-ThyTreg cells, pharmaceutical compositions, and combinations of the invention to a subject in need thereof.

### BRIEF DESCRIPTION OF THE DRAWINGS

FIG. 1. (A) Scheme of the CAR-41BB cDNA sequence. (B) Scheme of the CAR-41BB protein structure. (C) Scheme of the CAR-41BB-ThyTreg recognizing one biotinylated antibody specific of a cell surface marker. (D) full-length nucleotide sequence coding CAR41-BB. (h: hinge; tm: transmembrane; sd: signalling domain).
FIG. 2. (COMPARATIVE PURPOSE). (A) Scheme of the CARCD28 cDNA sequence. (B) Scheme of the CARCD28 protein structure. (C) full-length nucleotide sequence coding CARCD28. (h: hinge; tm: transmembrane; sd: signalling domain).
FIG. 3. (A) Scheme of the CAR-thyTreg recognizing target cells specifically through a biotinylated antibody. (B) Scheme of all co-culture conditions to test the CAR-thyTreg phenotype and function.
FIG. 4. (A) Dot plot of the CD4 and CD8 gated on living CELLTRACE violet positive target cells. (B) Ratio CD8:CD4 of the targets PBMC in the co-culture experiment with thyTreg NT or engineered thyTreg in combination with specific, non-specific, biotinylated and non-biotinylated antibodies.
FIG. 5. Represents the frequency of viability of target cells. (A) Frequency of CD8 viability, gated on total living target cells. (B) Frequency of CD8 viability, gated on total living target cells.
FIG. 6. Represents the viability and CAR expression after 8 days post-transduction. (A) Dot plots from flow cytometry analysis showing the size (FSC) and 7AAD (viability) of the cells, non-treated (NT) or genetically modified by viral vectors. (B) Dot plots from flow cytometry analysis showing the GFP and CAR expression (labelled with Atto665-biotin) of the cells, non-treated (NT) or genetically modified by viral vectors.
FIG. 7. Represents the frequency of the suppression of CD4+ T (dark grey) and CD8+ T (light grey) cell proliferation gated on total living target cells.

### DETAILED DESCRIPTION OF THE INVENTION

Unless defined otherwise, all technical and scientific terms herein have the same meaning as commonly understood. Any methods and materials similar or equivalent to those described herein can be used to test the present invention. However, the preferred materials and methods are described herein. It is also to be understood that the terminology herein describes particular embodiments only and is not intended to be limiting.

Throughout the present specification and the accompanying clauses, the words "comprise" and variations such as "comprises", "comprising" are to be interpreted inclusively. That is, these words are intended to convey the possible inclusion of other elements or integers not specifically recited, where the context allows. The word "comprise" also includes the term "consists of".

In a first aspect the present invention provides a CAR-ThyTreg cell.

The CARs of the invention can redirect the ThyTreg specificity and reactivity towards a cell associated with hyper-activation, autoimmune pathology or transplant rejection. Those cells express the antigen that will be recognized by the tagged biomolecules (e.g. monoclonal or polyclonal antibodies, aptamers, nanoparticles, proteins or peptides, and alike).

In the context of the invention, "Chimeric Antigen Receptor (CAR)" refers to a chimeric construct comprising an extracellular domain joined to a hinge, a transmembrane, and one or more intracellular signalling domains.

In one embodiment of the first aspect, the CAR-ThyTreg cell comprises the recombinant nucleic acid coding for the CAR41-BB SEQ ID NO: 7 or 8, or one having at least 85% of identity with SEQ ID NO: 7 or 8. In an alternative embodiment, the ThyTreg cell expresses on the surface a CAR amino acid sequence SEQ ID NO: 6 (CAR41-BB) or one having at least 85% of identity with SEQ ID NO: 6.

The term "identity" as used herein refers to an exact nucleotide-to-nucleotide or amino acid to amino acid correspondence of two polynucleotides or polypeptide sequences, respectively. Two or more sequences (polynucleotide or amino acid) can be compared by determining their "percent identity. The "percent identity" of two sequences, whether nucleic acid or amino acid sequences, is the number of exact matches between two aligned sequences divided by the length of the shorter sequence and multiplied by 100. Suitable programs for calculating the percent identity or similarity between sequences are well known in the art, such as the NCBI BLAST program, used for example with default parameters (http://www. ncbi. nlm. gov/cgi-bin/BLAST).

In one embodiment, the extracellular domain comprises a molecule from a pair of complementary affinity molecules. In some embodiments, a pair of complementary affinity molecules is selected from the group consisting of: biotin/biotin-binding moiety, antibody/antigen, enzyme/substrate, receptor/ligand, metal/metal-binding protein, carbohydrate/carbohydrate-binding protein, lipid/ a lipid-binding protein; and a His-tag/His-tag-binding molecule. In some embodiments, the pair of complementary affinity molecules is biotin/biotin-binding moiety. In this embodiment, the CAR-ThyTreg cells of the invention will not directly bind to the target but will require the interaction with the other member of the pair of specific binding moieties (in the examples below, a biotinylated intermediate) to stimulate the specific function of the CAR-ThyTreg cells.

In another embodiment, the extracellular domain includes a biotin-binding moiety. Illustrative non-limitative examples of biotin-binding moieties suitable in the context of the invention are rizavidin, avidin, streptavidin, bradavidin, tamavidin, lentiavidin, zebavidin, neutravidin, captavidin^{™}, combinations or functional fragments thereof. By "functional fragment" it is understood a fragment of the biotin-binding moiety which retains part or all the binding ability of the full-length moiety. In this embodiment, the CAR-ThyTreg cells of the invention will not bind to the target, but to biotinylated moieties (such as an antibody or fragment thereof, or aptamer, for instance) to stimulate the specific function of the CAR-ThyTreg cells.

In one embodiment, the extracellular domain comprises or consists of streptavidin of a fragment thereof.

Streptavidin is a 52.8 kDa protein from the bacterium *Streptomyces avidinii.* Streptavidin exists in nature as a homo-tetramer. The secondary structure of a streptavidin monomer is composed of eight antiparallel β-strands, which fold to give an antiparallel beta barrel tertiary structure. A biotin binding-site is located at one end of each β-barrel. Four identical streptavidin monomers (i.e. four identical β-barrels) associate to give streptavidin's tetrameric quaternary structure. The biotin binding-site in each barrel consists of residues from the interior of the barrel, together with a conserved Trp120 from neighbouring subunit. In this way, each subunit contributes to the binding site on the neighbouring subunit, and so the tetramer can also be considered a dimer of functional dimers. The streptavidin domain of the CAR system of the present invention may consist essentially of a streptavidin monomer, dimer, or tetramer. Particularly, it is a monomeric streptavidin having a sequence with an identity of at least 85%, at least 90% or at least 95% with respect to SEQ ID NO: 2 (high affinity monomeric streptavidin domain, known as mSA2, already disclosed in Lohmueller, J.J., *et al.,* 2017):
SEQ ID NO: 2

METDTLLLWVLLLWVPGSTGGAEAGITGTWYNQHGSTFTVTAGADGNLTGQYENRA
QGTGCQNSPYTLTGRYNGTKLEWRVEWNNSTENCHSRTEWRGQYQGGAEARINTQ
WNLTYEGGSGPATEQGQDTFTKVKPSAASGS

In another embodiment of the first aspect of the invention, the extracellular domain does not include neither is fused to an antibody or fragment thereof.

In one embodiment, the Chimeric Antigen Receptor (CAR) of the invention comprises an extracellular domain having a monomeric high-affinity streptavidin recognition domain, a hinge region, a transmembrane domain, and one or two cytoplasmic domains. In this embodiment, the extracellular domain does not include neither is fused to an antibody or fragment thereof.

The hinge region in CARs functions as a flexible spacer for the extracellular domain and improves the recognition/function of CARs for antigens closer located to the surface of target cells. For large number of constructs, the hinge region may consist of the CH₂CH₃ domain of a human IgG antibody, which can bind to Fc-receptors on macrophages and other cells, thereby resulting in cross-activation and activation-induced cell death *in vivo* independent of recognition of the target antigen. In one embodiment of the invention, the hinge region originates from CD8a (SEQ ID NO: 3):
SEQ ID NO: 3 TTTPAPRPPTPAPTIASQPLSLRPEACRPAAGGAVHTRGLDFACD

The CAR must include a transmembrane domain fused to the hinge and intracellular domains. The transmembrane domain can be derived from a natural or a synthetic source. When the transmembrane domain is from a natural source, the domain can be derived from any membrane-bound or transmembrane protein. For example, transmembrane domain from CD28, CD3 epsilon, CD45, CD4, CD5, CD8, CD9, CD16, CD22, CD33, CD37, CD154. Preferably, the transmembrane domain is the CD28 transmembrane domain having SEQ ID NO: 4: MFWVLVVVGGVLACYSLLVTVAFIIFWV.

In the context of the present invention, the term "Intracellular Domain" is the signal-transmission portion of a classical CAR. In the signalling system of the present invention the intracellular signalling domain (signalling domain) is located in the signalling component.

In the context of the present invention, the term "costimulation domain" refers to a signalling moiety that provides to T cells a signal which, in addition to the primary signal provided by, for instance, the CD3ζ chain of the TCR/CD3 complex, mediates a T cell response, including, but not limited to, activation, proliferation, differentiation, cytokine secretion, and the like. A co-stimulatory domain can include, in addition to 4-1BB (SEQ ID NO: 1), all or a portion of CD27, CD28, OX40 (CD134), CD30, CD40, ICOS, lymphocyte function-associated antigen-1 (LFA-1), CD2, CD7, LIGHT, NKG2C, B7-H3, and a ligand that specifically binds with CD83. In some embodiments, the costimulatory signalling domain is an intracellular signalling domain that interacts with other intracellular mediators to mediate a cell response including activation, proliferation, differentiation and cytokine secretion, and the like. In one embodiment of the present invention, the cytoplasmatic costimulation domain is CD3zeta (SEQ ID NO: 5): RVKFSRSADAPAYQQGQNQLYNELNLGRREEYDVLDKRRGRDPEMGGKPRRKNPQ EGLYNELQKDKMAEAYSEIGMKGERRRGKGHDGLYQGLSTATKDTYDALHMQALPPR

In one embodiment, the intracellular domain comprises the fused CD28-41-BB sequence referred as SEQ ID NO: 12 (SEQ ID NO: 12):

RSKRSRGGHSDYMNMTPRRPGPTRKHYQPYAPPRDFAAYRSGGGKRGRKKLLYIFK
QPFMRPVQTTQEEDGCSCRFPEEEEGGCEL.

In an alternative embodiment, the intracellular domain consists of 4-1BB sequence SEQ ID NO: 1.

In another embodiment of the first aspect of the invention, the CAR comprises (i) mSA2, (ii) CD8a hinge region, (iii) CD28 transmembrane region, (iv) SEQ ID NO: 1 or 7, and (v) CD3zeta, wherein (i) to (v) are provided in N-terminal to C-terminal order. In one embodiment, the CAR sequence is at least 85, 86, 87, 88, 89, 90, 91, 92, 93, 94, 95, 96, 97, 98, 99 or 100 % identical to SEQ ID NO: 6:
SEQ ID NO: 6:

METDTLLLWVLLLWVPGSTGGAEAGITGTWYNQHGSTFTVTAGADGNLTGQYENRA

QGTGCQNSPYTLTGRYNGTKLEWRVEWNNSTENCHSRTEWRGQYQGGAEARINTQ
WNLTYEGGSGPATEQGQDTFTKVKPSAASGSTTTPAPRPPTPAPTIASQPLSLRPEA
CRPAAGGAVHTRGLDFACDMFWVLVVVGGVLACYSLLVTVAFIIFWVKRGRKKLLYIF
KQPFMRPVQTTQEEDGCSCRFPEEEEGGCELRVKFSRSADAPAYQQGQNQLYNEL
NLGRREEYDVLDKRRGRDPEMGGKPRRKNPQEGLYNELQKDKMAEAYSEIGMKGE
RRRGKGHDGLYQGLSTATKDTYDALHMQALPPR

The nucleic acid coding to the CAR of the invention is a sequence with at least 85, 86, 87, 88, 89, 90, 91, 92, 93, 94, 95, 96, 97, 98, 99 or 100 % of identity with SEQ ID NO: 7:

GGCGCAGAGGCGGGTATCACCGGGACATGGTACAACCAACACGGAAGCACATTT
ACAGTCACCGCTGGAGCAGACGGGAATCTGACCGGACAGTACGAGAACAGGGCT
CAGGGGACAGGTTGTCAGAACAGTCCGTATACTCTGACTGGGAGGTACAATGGC
ACGAAGCTGGAGTGGCGAGTCGAGTGGAATAATTCCACGGAAAACTGTCACAGTA
GAACAGAGTGGAGGGGACAGTACCAGGGGGGAGCAGAGGCCCGGATCAACACC
CAATGGAACTTGACATATGAAGGCGGGTCAGGCCCCGCGACAGAGCAAGGACAG
GATACATTCACGAAGGTCAAGCCAAGCGCAGCCTCTGGCTCTACCACAACTCCAG
CTCCCCGGCCCCCTACTCCTGCTCCAACCATTGCCTCACAGCCACTGAGCCTGC
GGCCCGAAGCTTGTAGACCTGCTGCTGGAGGAGCTGTGCATACCAGAGGCCTGG
ACTTCGCCTGCGATATGTTCTGGGTGCTGGTGGTGGGCGGGGTGCTGGCCT
GCTACAGCCTGCTGGTGACAGTGGCCTTCATCATCTTTTGGGTGAAACGGGGCAG
AAAGAAACTCCTGTATATATTCAAACAACCATTTATGAGACCAGTACAAACTACTCA
AGAGGAAGATGGCTGTAGCTGCCGATTTCCAGAAGAAGAAGAAGGAGGATGTGA
ACTGCGGGTGAAGTTCAGCAGAAGCGCCGACGCCCCTGCCTACCAGCAGGGCCA
GAATCAGCTGTACAACGAGCTGAACCTGGGCAGAAGGGAAGAGTACGACGTCCT
GGATAAGCGGAGAGGCCGGGACCCTGAGATGGGCGGCAAGCCTCGGCGGAAGA
ACCCCCAGGAAGGCCTGTATAACGAACTGCAGAAAGACAAGATGGCCGAGGCCT
ACAGCGAGATCGGCATGAAGGGCGAGCGGAGGCGGGGCAAGGGCCACGACGG
CCTGTATCAGGGCCTGTCCACCGCCACCAAGGATACCTACGACGCCCTGCACAT
GCAGGCCCTGCCCCCAAGG

Before expansion and genetic modification of the regulatory T cells of the invention, a source of ThyTreg cells is obtained from a subject.

Preferably, the Treg cells suitable in the context of the invention are characterized by comprising CD4+CD8+FOXP3. A population with these suitable Treg cells can be obtained following the method disclosed in EP3759215A1, whose content is incorporated herein by reference. The resulting cells have a great capacity to suppress effector cells of the immune system, an undifferentiated (naive) phenotype, high viability, and a stable expression of FOXP3 even under pro-inflammatory conditions and functional markers such as CTLA-4 and CD39.

In one embodiment, thymic Treg cells are obtained following the steps:
a. mechanically disaggregating an isolated thymic tissue;
b. filtering the product obtained after stage (a), and resuspending the precipitate comprising thymocytes in a culture medium in the presence of IL-2;
c. isolating CD25+ cells from the product obtained after stage (b);
d. culturing the cell population obtained after stage (c) in a culture medium in the presence of a T cell activator and IL-2, wherein said T cell activator comprises at least CD3 and CD28 agonists; and
e. removing the T cell activator from the culture medium of stage (d);
f. optionally, further culturing the regulatory T cells in a culture medium in the presence of IL-2;
with the proviso that prior to step (d) the cell population has not been depleted from CD8+ cells.

The thyTreg cells resulting from the above isolation method are characterized by a higher level of CD27, CD45RA, and CCR4; as well as a lower amount of activated HLA-DR, the latter reducing their immunogenicity.

In the context of the invention, the "thymic tissue" is any tissue sample from the thymus, which is the gland of the lymphatic system where T cells or lymphocytes mature, located in front of the heart and behind the breastbone. The thymic tissue can be removed by any method known in the art that serves such purpose, such as for example by means of a thymectomy, which can be transsternal, transcervical or videoscopic. Preferably, the thymic tissue is removed, prior to carrying out the method, during a surgical intervention, more preferably intended for treating a heart disease, such as for example congenital heart disease, or during a heart transplant. Even more preferably, the thymic tissue of the invention is removed during a paediatric heart transplant.

The thymic tissue can come from a human or a non-human mammal such as, for example, but not limited to, rodents, pigs, primates, ruminants, felines or canines. In a preferred embodiment of the method of the invention, the thymic tissue comes from a human, more preferably a human aged between 0 (newborn) and 16 years, even more preferably between 0 and 10 years, particularly between 0 and 24 months.

In another preferred embodiment, the thymic tissue comes from the same or different individual to whom the ThyTreg cells obtained at the end of the method of the invention are going to be subsequently administered for cell immunotherapy.

In the present invention, "paediatric patient" or "child" is understood to be a human aged between 0 and 16 years, preferably between 0 and 10 years, even more preferably between 0 and 24 months.

Any tissue dissociator from among those commercially available in the state of the art could be used in stage (a) of the method of the invention. Examples of these dissociators are, but not limited to, the gentleMACS Dissociator or the gentleMACS Octo Dissociator from Miltenyi Biotec, the TissueLyser LT from Qiagen or tissue dissociators from Worthington Biochemical, Sigma-Aldrich or Roche Diagnostics. Preferably, the tissue dissociator used in the present invention is the gentleMACS Octo Dissociator from Miltenyi Biotec in one embodiment, step a) comprises mechanically disaggregating the thymic tissue in the presence of a culture medium and without using enzymes.

Whether before or after genetic modification of the Treg cells to express a desirable CAR, the T cells can be activated and expanded. Generally, the T cells of the invention are expanded by contact with a surface having attached thereto an agent that stimulates a CD3/TCR complex associated signal and a ligand that stimulates a co-stimulatory molecule on the surface of the T cells. In the invention, once ThyTreg are freshly isolated from the thymus, and after verification of their purity by flow cytometry (CD25+FOXP3+), cells can be activated and expanded with anti-CD3/anti-CD28 magnetic beads (Dynabeads^{™}) at a ratio of 1:1 (bead : cell). In one embodiment, the ratio of CD3:CD28 antibody bound to the beads ranges from 100:1 to 1:100 and all integer values there between. In one embodiment, the cells can be re-activated and re-stimulated for expansion after 7 days after the first activation and expansion. In this embodiment the re-activation and re-stimulation can be done with anti-CD3/anti-CD28 magnetic beads (Dynabeads^{™}) or anti-CD3/anti-CD28 matrix (Miltenyi Biotec^{™}). The ratio between the matrix and the cells ranges from 1:50 to 1:1000, with all integer values between. In one embodiment, the cells (10⁴ to 10⁹ ThyTreg cells) and matrices or beads are combined in culture medium, preferably X-vivo15 or PBS (without divalent cations such as calcium and magnesium). Those of ordinary skill in the art can readily appreciate any cell concentration that may be used. For example, a concentration of about 2 million cells/ml is used in one embodiment. In another embodiment, a concentration of cells of 10, 15, 20, 25, 30, 35, 40, 45, 50 or 100 million cells/ml is used. Using high concentrations can result in increased cell yield, cell activation, and cell expansion.

In one embodiment, the mixture may be cultured for several hours (about 18 hours) to about 72 hours or any hourly integer value in between. Several cycles of stimulation/expansion may also be desired such that the culture time of T cells can be 28 days or more.

Conditions appropriate for T cell culture include an appropriate media, preferably X-Vivo 15 (Lonza), but also Minimal Essential Media or RPMI Media1640 (Lonza) that may contain factors necessary for proliferation and viability, including serum (e.g., fetal bovine or human serum), interleukin-2 (IL-2), IL-7, or IL-15.

Other additives for the growth of cells include, but are not limited to, reducing agents such as 2-mercaptoethanol. Media can include RPMI 1640, AIM-V, DMEM, MEM, C-MEM, F-12, X-Vivo 15, and X-Vivo 20, Optimizer, with added amino acids, sodium pyruvate, and vitamins, either serum-free or Supplemented with an appropriate amount of serum (or plasma), and/or an amount of cytokine(s) sufficient for the growth and expansion of T cells. The target cells are maintained under conditions necessary to support growth, for example, an appropriate temperature (e.g., 37°C.) and atmosphere (e.g., air plus 5% CO).

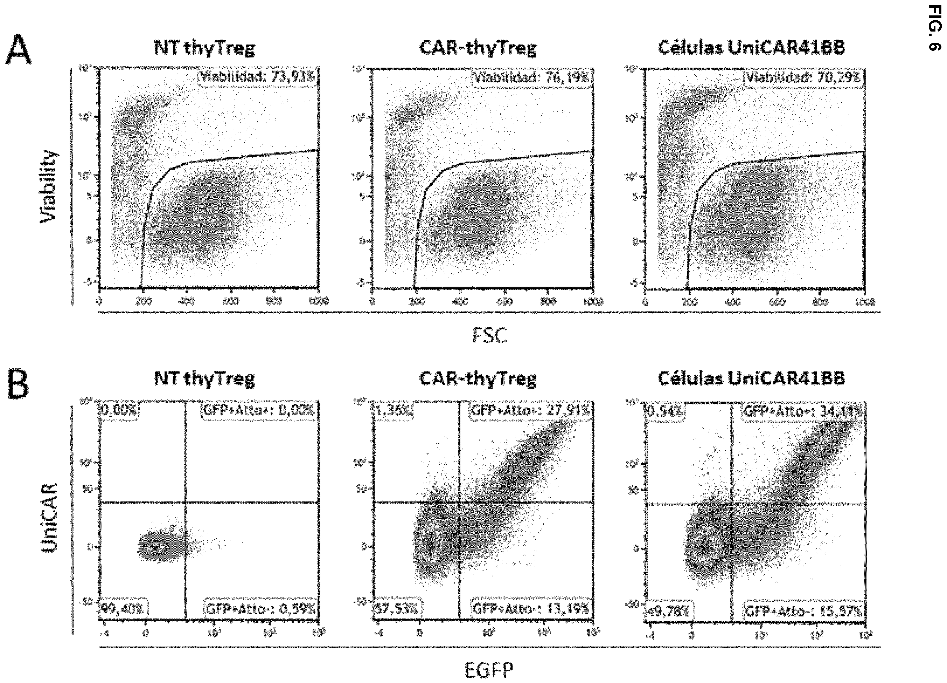

24h after activation and without removing activation beads, viral vectors coding for our CAR are added to the cell mixture. 24h after lentivector treatment (in one embodiment, 48 hours after lentivector treatment), magnetic beads are removed with a magnet. Cells are then washed with a warm culture medium, preferably X-vivo15 complemented with 5% human serum, and tested for viability with the 7-Aminoactinomycin D (7-AAD, Figure 6A) and GFP expression by flow cytometry. The transduction frequency is around 40% (from 20 to 70%, Figure 6B). If cells presented less than 30% of transduction, GFP+ cells are sorted by the Tyto sorter (Miltenyi Biotec). Cells are then let to proliferate for 5 days adding fresh medium RPMI 1640 complemented with 5% human serum (Lonza) and recombinant human IL-2 (600 U/ml, preferably Immunotools). After 8 days, cells are labelled with a biotinylated marker (preferably Atto665-biotin, Sigma-Aldrich) for CAR detection (Figure 6B). Since the CAR plasmids encode mSA2 and GFP, the double-positive cells were well-transduced and expressed GFP and CAR.

In one embodiment, the isolation of CD25+ cells in step c) comprises the use of magnetic beads conjugated to antibodies against CD25.

In one embodiment, the T cell activator of stage d) is a colloidal polymer nanomatrix (magnetic beads) conjugated with humanised CD3 and CD28 agonists. These magnetic beads facilitate the efficient activation of T cells while maintaining the viability thereof.

In one embodiment, the T-cell activator of stage d) is used in a T-cell activator: cell ratio range between 1:10 and 1:100.

In one embodiment, the cells are cultured in step d) for at least 2 or 3 days, preferably for at least 3 days.

In one embodiment, the cells are cultured in step d) in the absence of rapamycin.

In one embodiment, the removal of stage e) is carried out by means of centrifugation or magnet.

In one embodiment, in step f) the regulatory T cells are cultured for another one to seven days, preferably for another four days in the presence of IL-2.

In one embodiment, the cells are cultured in step f) in the absence of rapamycin.

In one embodiment, said culture medium is a GMP culture medium.

In one embodiment, the culture medium further comprises antibiotics, preferably 5% of antibiotics.

The CAR-ThyTreg cells of the invention can be generated by introducing an expression construct comprising the CAR as defined in any of the embodiments provided above. In one embodiment, the expression construct comprises a sequence with at least 85%, at least 90% or at least 95% of identity with respect to sequence SEQ ID NO: 7.

The nucleic acid CAR sequence can be cloned into many types of vectors. For example, the nucleic acid can be cloned into a vector including, but not limited to, a plasmid, a phagemid, a phage derivative, an animal virus, and cosmid. Further, the expression vector may be provided to a cell as a viral vector. Viral vector technology is well-known in the art. Viruses useful as vectors include, but are not limited to, retroviruses, adenoviruses, adeno-associated viruses, herpes viruses, and lentiviruses. Generally, a suitable vector contains an origin of replication functional in at least one organism, a promoter sequence, convenient restriction endonuclease sites, and one or more selectable markers.

The method of the present invention uses a DNA construct comprising a nucleic acid coding for the CAR as defined in any of the above embodiments. In one embodiment, the expression vector derives from a lentivirus. Lentivirus are suitable tools for long-term gene transfer since they allow long-term, stable transgene integration and its propagation in daughter cells. Lentiviral vectors have the advantage over other vector sources: they can transduce non-proliferating cells and have low immunogenicity.

The expression of the nucleic acid coding for the CAR object of the invention is typically achieved by operably linking a nucleic acid encoding the CAR polypeptide or portions thereof to a promoter and incorporating the construct into an expression vector. Typical cloning vectors contain transcription and translation terminators, initiation sequences, and promoters helpful in regulating the expression of the desired nucleic acid sequence.

Illustrative non-limitative examples of promoter sequences are the cytomegalovirus (CMV), simian virus 40 (SV40) early promoter, mouse mammary tumour virus (MMTV), human immunodeficiency virus (HIV) long terminal repeat (LTR) promoter, MoMul V promoter, an avian leukaemia virus promoter, an Epstein-Barr virus immediate early promoter, a Rous sarcoma virus promoter, as well as human gene promoters such as but not limited to, the actin promoter, the myosin promoter, the haemoglobin promoter, human elongation factor 1 alpha (EF1a), and the creatine kinase promoter. Further, the invention should not be limited to constitutive promoters. Inducible promoters are also contemplated as part of the invention. Using an inducible promoter provides a molecular switch capable of turning on the expression of the polynucleotide sequence, which is operatively linked when such expression is desired or turning off the expression when the expression is not desired.

In the context of the present invention, the terms "operably linked", "operably linking", refers to a functional linkage between a regulatory sequence and a heterologous nucleic acid sequence resulting in the expression of the later. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions in the same reading frame.

To assess the expression of a CAR polypeptide or portions thereof, the expression vector to be introduced into a cell can also contain either a selectable marker gene or a reporter gene or both to facilitate the identification and selection of expressing cells from the population of cells sought to be transfected or transduced through viral vectors. Both, selectable markers and reporter genes may be flanked with appropriate regulatory sequences to enable expression in the host cells.

Reporter genes identify potentially transduced cells and evaluate regulatory sequences' functionality. Expression of the reporter gene is assayed at a suitable time after the DNA has been introduced into the recipient cells. The reporter gene can be coded in the same reading frame as the CAR sequences, separated by a T2A sequence, the 2A peptide from *Thosea asigna* virus capsid protein; or, alternatively, it can be under a promoter that can be different from the CAR promoter.

In another embodiment, the expression construct consists of a sequence with at least 85%, at least 90%, or at least 95% of identity with respect to sequence SEQ ID NO: 8.

Methods of introducing and expressing genes into a cell are known in the art. In the context of an expression vector, the vector can be readily introduced into a host cell, e.g., mammalian, bacterial, yeast, or insect cell, by any method in the art. Biological methods for introducing a polynucleotide of interest into a host cell include DNA and RNA vectors. Viral vectors, especially retroviral vectors, have become one of the most widely used methods for inserting genes into mammals, e.g., human cells. Other viral vectors can be derived from lentivirus, poxviruses, herpes simplex virus I, adenoviruses and adeno-associated viruses, and the like. Regardless of the method used to introduce exogenous nucleic acids into a host cell, various assays may be performed to confirm the presence of the recombinant DNA sequence in the host cell. Such assays include, for example, "molecular biological' assays well known to those of skill in the art, such as Southern and Northern blotting, RT-PCR and PCR; "biochemical assays, such as detecting the presence or absence of a particular peptide, e.g., by immunological means (ELISA, flow cytometry and Western blots) or by assays described herein to identify agents falling within the scope of the invention.

In a further aspect the present invention provides CAR-ThyTreg cells obtainable by the process of the invention.

The cells resulting from the above process are characterized by a high expression, among others, of perforin, granzyme B or both. Preferably, the cells show overexpression of both perforin and granzyme B.

For the purposes of the invention the expressions "obtainable", "obtained" and equivalent expressions are used interchangeably, and in any case, the expression "obtainable" encompasses the expression "obtained".

The present invention is based on improving the immunosuppressive ThyTreg ability in modulating immune system status in hyper-inflammation processes, autoimmune diseases, or transplant rejection, among others.

Accordingly, the invention provides CAR-ThyTreg cells and methods of their use for adoptive therapy. In one embodiment, the CAR-ThyTreg cells of the invention can be generated by introducing a lentiviral vector comprising a desired CAR, for example, a CAR comprising anti-high affinity monomeric streptavidin, CD8alpha hinge, a CD28 transmembrane domain, and human 41-BB and the CD3zeta signalling domains, into the cells. The CAR-ThyTreg cells of the invention can replicate *in vivo* resulting in long-term persistence that can lead to sustained immunosuppressive control.

In one embodiment, the invention relates to administering a genetically modified ThyTreg cell expressing a CAR for treating a patient with hyper-inflammation processes, autoimmune diseases, or transplant rejection. The CAR-ThyTreg could be autologous or allogeneic. The ThyTreg can be isolated from human thymus from pediatric patients undergoing cardiac surgery.

In a further aspect the present invention provides a combination comprising a ThyTreg cell population as defined in any of the previous embodiments, incorporating a biotin-binding moiety; and an activated immune cell specific-binding moiety, which is conjugated to biotin.

All the embodiments provided under the first aspect of the invention, related to the biotin-binding moieties and CAR sequences included thereof, are also embodiments of the combination of the invention.

The "activated immune cell specific-binding moiety" can be, in the context of the invention, any molecule with the ability to bind to an immune cell. The term "immune cell" as used herein includes any cell that is involved in the generation, regulation, or effect of the acquired or innate immune system. Immune cells include T cells such as CD4+ cells, CD8+ cells and various other T cell subsets, B cells, natural killer cells, macrophages, monocytes and dendritic cells, and neutrophils. Thus, the "activated immune cell specific-binding moiety" can be an antibody, aptamer, cytokine, any nucleic or peptide sequence, or fragment thereof with the ability to bind to a particular component (marker) related to the activation of the immune cell. Illustrative non-limitative examples of surface markers already known in the state of the art as being specific of activated immune cells are CD19, CD30, CD8, and the interleukin 23 receptor, among others.

In the context of the invention the term "antibody", refers to an immunoglobulin molecule which specifically binds with an antigen. Illustrative non-limitative examples of "antigen" in the context of the invention are such as, but not limited to, CD30, HLA-A2, CD19, Insulin, 2,4,6-trinitrophenol (TNP), Carcinoembryonic antigen (CEA), Myelinoligodendrocyte glycoprotein (MOG), Myelin basic protein (MBP), B cell maturation antigen (BCMA), or Desmosomal core glycoprotein-3 (DSG3).

Antibodies can be intact immunoglobulins derived from natural or recombinant sources and immunoreactive portions of intact immunoglobulins. Antibodies may exist in various forms, including, for example, polyclonal antibodies, monoclonal antibodies, multispecific antibodies such as bispecific antibodies, humanised antibodies, and fragment antibodies, as long as they exhibit the desired target-binding activity. Non-limiting examples of antibody fragments are Fv, Fab, Fab, Fab-SH, and F(ab)2, diabodies, linear antibodies, single chain antibodies and multispecific antibodies formed from antibody fragments.

In one embodiment, the mSA2-CAR protein can be designed to target biotinylated aptamers.

In the context of the invention, the term "aptamer" refers to an oligonucleotide (nucleic acid aptamer) or peptide molecule that binds to a specific target molecule. Nucleic acid aptamers are sequences engineered through repeated selection rounds to bind to various molecular targets such as small molecules, proteins, nucleic acids, cells, or tissues.

In one embodiment, the mSA2-CAR can be designed to target biotinylated cytokines.

In one embodiment, the mSA2-CAR can be designed to target biotinylated receptor ligands.

In one embodiment, the mSA2-CAR can be designed to target a biotinylated nucleic acid sequence.

By "biotinylation" in biochemistry is the process of covalently attaching biotin to a protein, nucleic acid, or another biomolecule. There are well-known protocols in the state of the art to biotinylate the molecule of interest.

However, the invention should not be construed as limited solely to the targets and diseases disclosed herein. Instead, the invention should be construed to include any antigenic target associated with a disease where a CAR can be suitable to be used.

The CAR-modified T cells of the present invention may be administered either alone or as a pharmaceutical composition in combination with one or more pharmaceutical acceptable excipients or carriers.

As used herein, "pharmaceutically acceptable excipient", or "pharmaceutically acceptable carrier" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as urea, glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone.

In the context of the invention, the term "therapeutically effective amount" refers to the amount of the subject compound that will elicit the biological or medical response of a tissue, system, or subject being sought by the researcher, veterinarian, medical doctor, or other clinicians. The term "therapeutically effective amount" includes the amount of a compound that, when administered, is sufficient to prevent the development of, or alleviate to some extent, one or more of the signs or symptoms of the disorder or disease being treated. The therapeutically effective amount will vary depending on the compound, the disease and its severity and the age, weight, etc., of the subject to be treated. The dose for obtaining a therapeutically effective number depends on a variety of factors such as example, age, weight, sex, pathological condition, or tolerance of the individual to whom the composition of the invention is going to be administered. Preferably, the therapeutically effective amount of the invention comprised in the composition of the invention is between of 10⁴ to 10⁹ cells/kg body weight, preferably 10⁵ to 10⁷ cells/kg body weight, including all integer values within those ranges. T cell compositions may also be administered multiple times at these dosages.

The compositions described herein may be administered to a patient subcutaneously, intradermally, intracranial, intranodally, intramedullary, by intravenous (i.v.) injection, or intraperitoneally. The CAR-ThyTreg cell compositions of the present invention are preferably administered by i.v. injection.

In certain embodiments of the present invention, cells activated, expanded, and engineered using the methods described herein, or other methods known in the art where T cells are expanded to therapeutic levels, are administered to a patient in conjunction with (e.g., before, simultaneously or following) any biomolecules biotinylated that correspond to the therapeutic necessity. The medicament of the invention can be used both alone and in combination with other drugs or compositions for immune tolerance induction or for treating and/or preventing a pathological condition selected from the list consisting of autoimmune disease, inflammatory processes, allergy, graft-versus-host disease and/or immune rejection in transplanted individuals. These other medicaments or compositions to be administered as therapy combined with the medicament of the invention can form part of the same composition or can be administered utilizing different compositions and administered simultaneously with the medicament of the invention or at different times. The dosage of the above treatments to be administered to a patient will vary with the precise nature of the condition and the treatment recipient. The scaling of dosages for human administration can be performed according to art-accepted practices.

In a more preferred embodiment, the medicament of the invention is administered once a previous therapy with immunosuppressive drugs has been completed. At the moment of administering the medicament of the invention, the administration of immunosuppressive drugs can be eliminated to give way to the exclusive treatment with the medicament of the invention, or the dose of said immunosuppressive drugs can be gradually decreased until wholly eliminated.

In the context of the invention, "combination therapy", "in combination with" or "in conjunction with" as used herein denotes any form of concurrent, parallel, simultaneous, sequential, or intermittent treatment with at least two distinct treatment modalities (i.e., compounds, components, targeted agents or therapeutic agents). As such, the terms refer to administration of one treatment modality before, during, or after administration of the other treatment modality to the subject. The modalities in combination can be administered in any order. The therapeutically active modalities are administered together (e.g., simultaneously in the same or separate compositions, formulations, or unit dosage forms) or separately (e.g., on the same day or on different days and in any order as according to an appropriate dosing protocol for the separate compositions, formulations, or unit dosage forms) in a manner and dosing regimen prescribed by a medical caretaker or according to a regulatory agency. In general, each treatment modality will be administered at a dose and/or on a time schedule determined for that treatment modality. Optionally, three or more modalities may be used in a combination therapy. Additionally, the combination therapies provided herein may be used in conjunction with other types of treatment. For example, other anti-cancer treatment may be selected from the group consisting of chemotherapy, surgery, radiotherapy (radiation) and/or hormone therapy, amongst other treatments associated with the current standard of care for the subject.

Another aspect of the invention relates to a method for inducing or restoring immune tolerance in an individual, more preferably in a transplanted or grafted individual, or who has an autoimmune disease, or in an inflammatory process or an allergy or graft-versus-host disease, wherein said method comprises the administration of the CAR-ThyTreg cell, or the pharmaceutical composition or the combination, to said individual.

Another aspect of the invention relates to a method for treating and/or preventing a pathological condition selected from the list consisting of autoimmune disease, inflammatory process, allergy, graft-versus-host disease and/or immune rejection to a transplant, wherein said method comprises the administration of the CAR-ThyTreg cell of the invention or the pharmaceutical composition of the invention to an individual suffering from a said pathological condition.

"Autoimmune disease" is a disorder resulting from an autoimmune response which results from an inappropriate and excessive response to a self-antigen or autoantigen. Examples of "autoimmune diseases" that could be treated and/or prevented with the medicament of the invention are, but not limited to, type I diabetes, arthritis (such as, for example, rheumatoid arthritis, psoriatic arthritis, ankylosing spondylitis or juvenile idiopathic arthritis), multiple sclerosis, intestinal inflammatory affection of autoimmune origin (such as Chron's Disease or ulcerative colitis), vasculitis (such as Wegener's Disease or atherosclerosis), asthma, inflammatory autoimmune affection of the bile duct (such as primary biliary cirrhosis or primary sclerosing cholangitis), autoimmune thyroiditis (Hashimoto's Disease), hyperthyroidism (Graves's Disease), autoimmune adrenal insufficiency (Addison's Disease), autoimmune oophoritis, autoimmune orchitis, autoimmune hepatitis, autoimmune haemolytic anaemia, paroxysmal cold haemoglobinuria, autoimmune thrombocytopenia, autoimmune neutropenia, pernicious anaemia, pure red cell aplasia, autoimmune coagulopathies, myasthenia gravis, autoimmune polyneuritis, pemphigus and other blistering disorders, rheumatic heart disease, Goodpasture Syndrome, postcardiotomy syndrome, lupus erythematosus, Sjögren Syndrome, polymyositis, dermatomyositis, sclerodermia, chronic obstructive pulmonary diseases, chronic inflammatory diseases, celiac disease, Churg-Strauss Syndrome, cardiovascular disease, polydermatomyositis, septic shock, rhinitis, psoriasis, cancer-associated cachexia, eczema, Vitiligo, Reiter Syndrome, Kawasaki's Disease, idiopathic thrombocytopenic purpura, Guillain-Barré Syndrome, antiphospholipid antibody syndrome (APS) or narcolepsy.

In one embodiment, CAR-ThyTreg cells of the invention, when they include a member of a pair of members (such as mSA2), any of the therapeutic uses provided in the present invention will include administering an antibody, peptides or aptamer which is tagged with a member of the pair of specific binding moieties (such as biotin); and administering the CAR-ThyTreg or pharmaceutical composition of the invention, both steps being performed in any order (simultaneously or sequentially).

### EXAMPLES

The invention is further described in detail by reference to the following experimental example. This section is provided for illustration only and is not intended to be limiting unless otherwise specified. Thus, the invention should in no way be construed as being limited to the following example but, rather, should be construed to encompass any variations that become evident because of the instruction provided herein. Without further description, it is believed that using the preceding description and the following illustrative examples, make and utilize the compounds of the present invention and practice the claimed methods. The following working examples, therefore, specifically point out the preferred embodiments of the present invention and are not to be construed as limiting in any way the remainder of the disclosure.

New CAR-ThyTreg were generated with potent activity composed of the highly functional and naive ThyTreg engineered with high-affinity monomeric streptavidin (mSA2) protein for biotin. Higher affinity can lead to greater ThyTreg activity and response. It was determined that biotinylated antibodies and the presence of target cells efficiently stimulate mSA2-ThyTreg cells. Moreover, they were capable of potent suppressive function associated with direct target cell lysis.

### Materials and methods

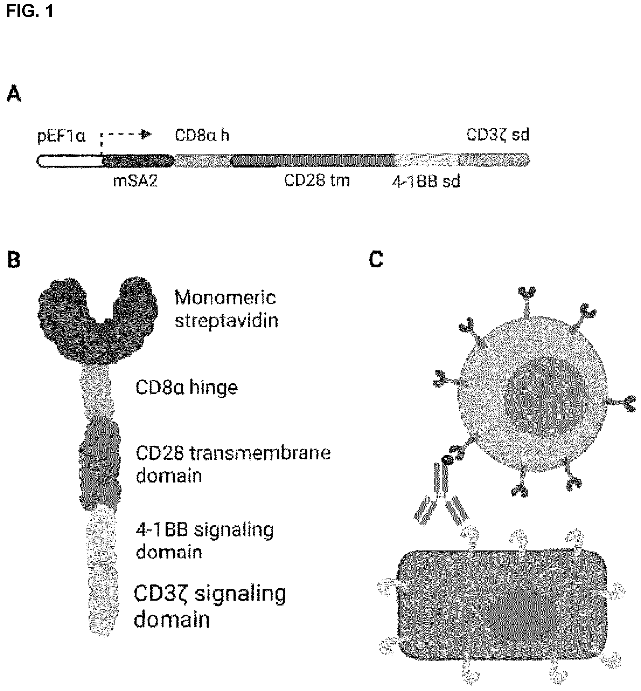

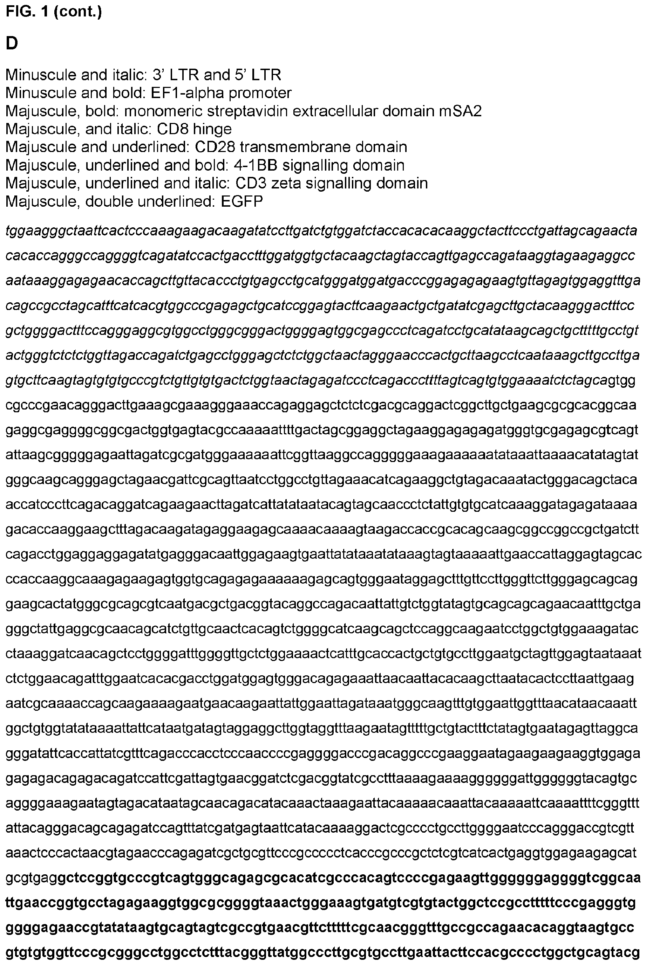

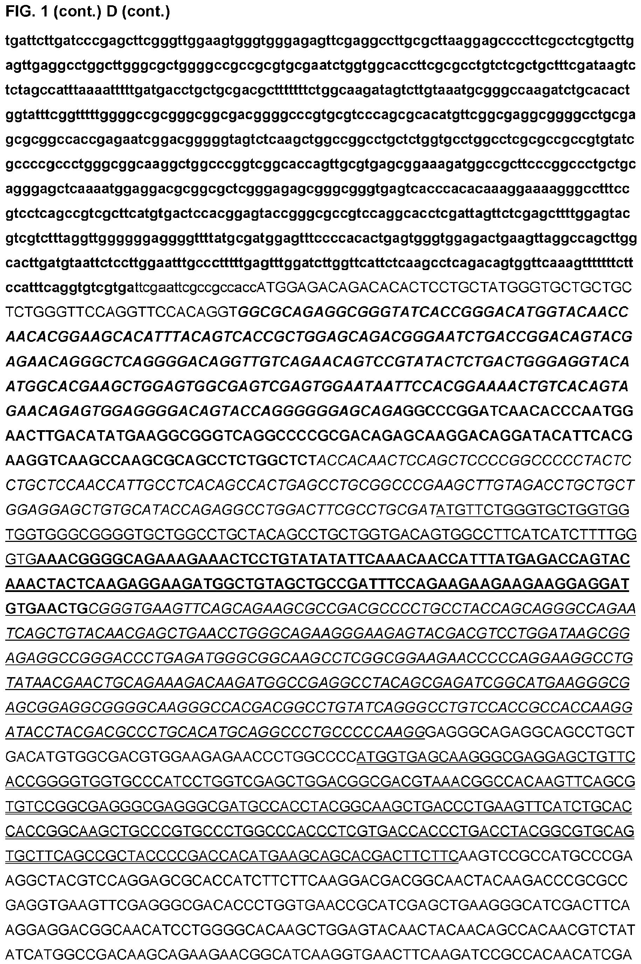

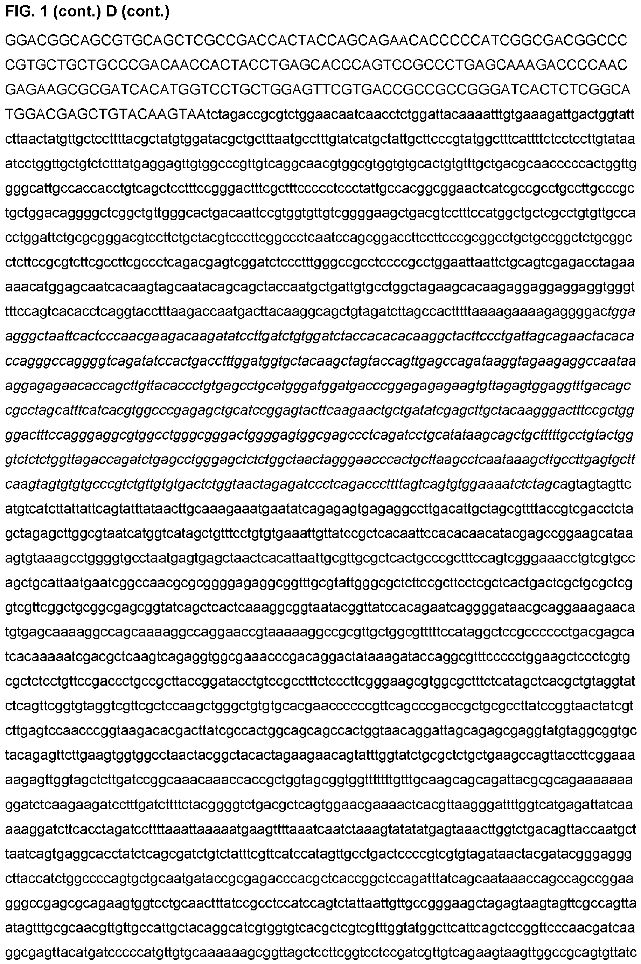

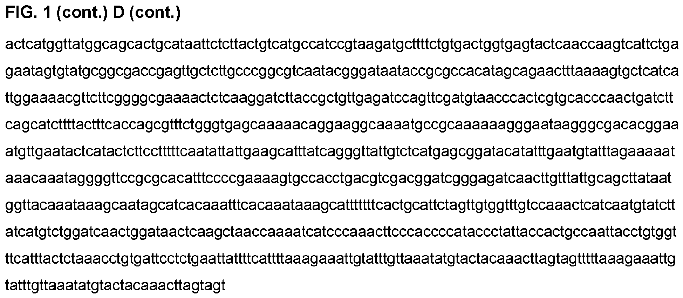

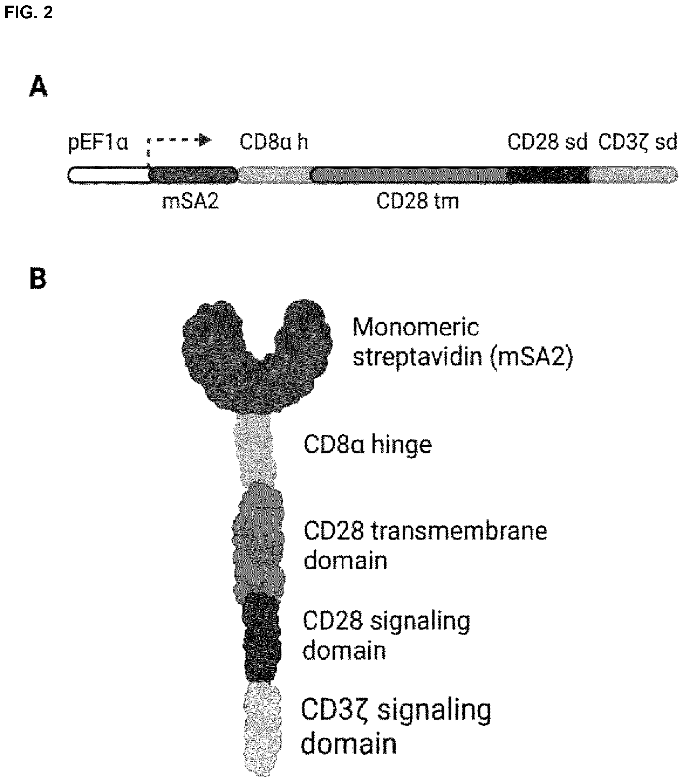

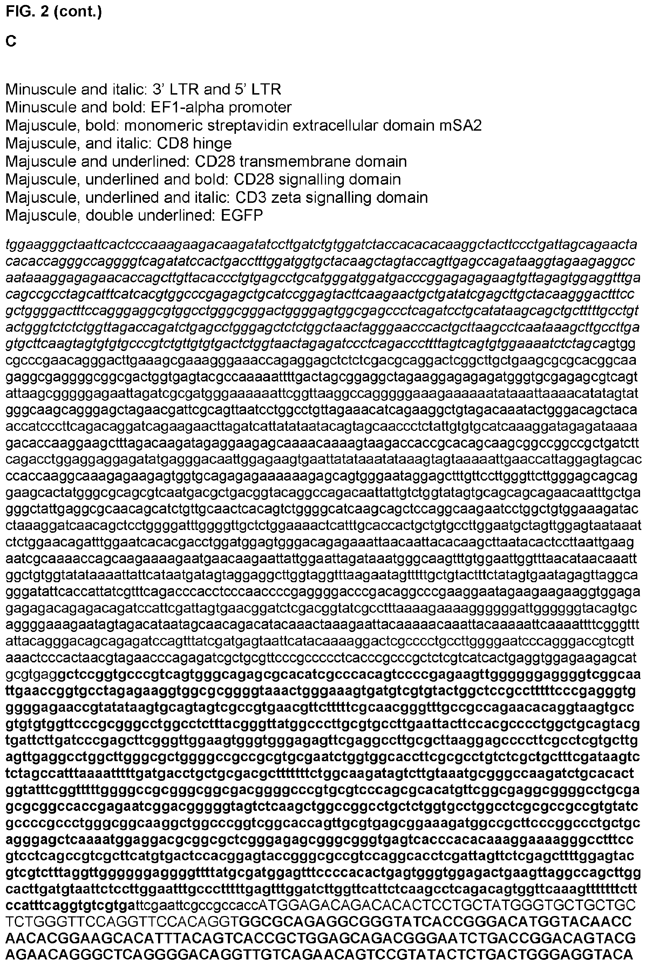

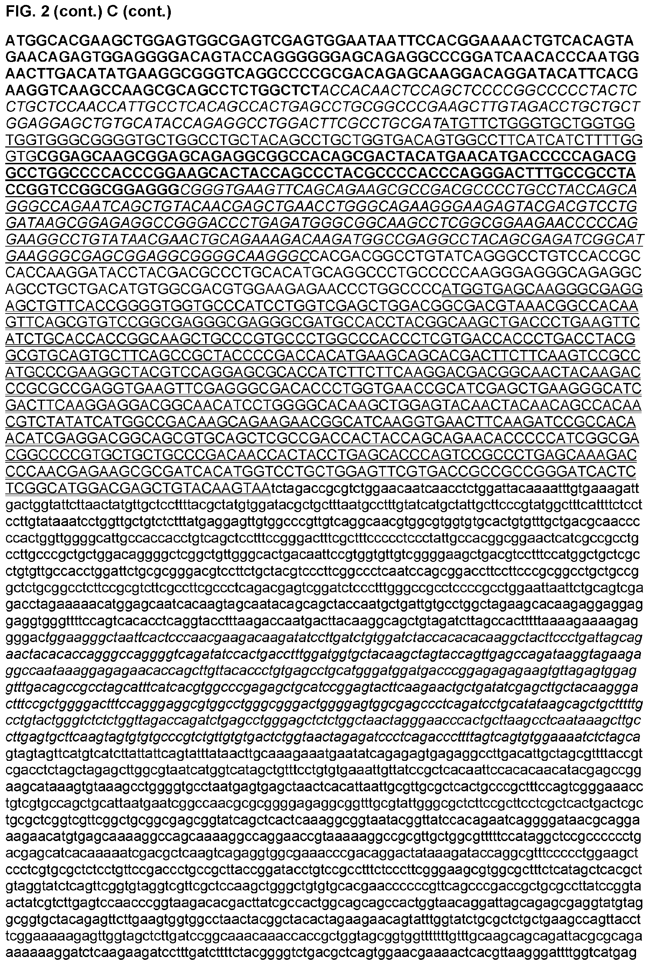

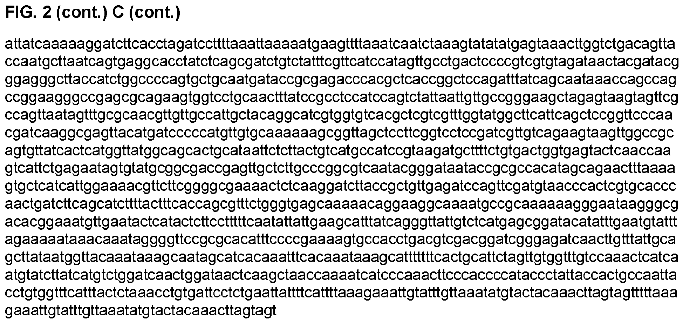

Lentivector constructs and lentivector production: lentiviral vector encoding for CAR (mSA2, SEQ ID NO: 2) coding regions were listed in FIG. 1 (CAR41-BB-ThyTreg), and FIG. 2 (CARCD28, for comparative purpose), and were synthesized (Creative Biolabs) and cloned into Lenti-EF1a (Creative Biolabs). The viral vector was generated using the psPAX2 and pMD2.G (both plasmids from Addgene, gift from Didier Trono Addgene plasmid # 12260 y # 12259, respectively). All plasmids were transfected into 293T cells (ref # CRL-3216 from ATCC) using the lentivector production protocol (Salmon and Trono, 2006). Lentiviral vectors were produced by the co-transfection of 293T cells (ATCC, LGC Standards S.L.U., Barcelona, Spain) with pCAR, psPAX2, and pMD2.G using a calcium phosphate transfection kit (Sigma-Aldrich, St. Louis, MO, USA). The physical titers of the vectors were evaluated after 0.45 µm filtration (Corning, Corning, NY, USA) by quantifying HIV-1-p24gag with an ELISA kit (Abcam, Cambridge, UK). In brief, on day one, one million 293T cells were plated in a 10 cm dish. On day two, the cells were transfected with three plasmids using a calcium phosphate-DNA precipitate method. The plasmids were mixed in sterile water to a final concentration of 1-10 µg/µl and added dropwise to 2.5 M CaCl2 solution mixed with HBS. After incubating for 20-30 minutes, the DNA-CaCl2-HBS mixture was added dropwise to the cells and incubated for 18 hours at 37°C. The cells were then rinsed and cultured for 24-72 hours before proceeding to lentivector filtration and storage at -80°C.

Biotinylated Antibodies: anti-CD8 (#REA734, Miltenyi Biotec), Isotype control, rh-lgG1 (#REA293, Miltenyi Biotec), and anti-CD8alpha (#OKT8, Creative Biolabs) were all purchased already biotinylated. The antibody anti-CD8 (#REA734, Miltenyi Biotec) non-biotinylated was also purchased as control of the experiments.

Cell lines: Human cell lines 293T for lentivector production (# CRL-3216) were cultured at 37°C. 293T cells were cultured in RPMI medium supplemented with 1% mix of antibiotics (125 µg/mL ampicillin, 125 µg/mL cloxacillin, and 40 µg/mL gentamicin) and 5% of fetal bovine serum (FBS).

Primary peripheral blood mononuclear cells (PBMC). PBMC were isolated from de-identified human buffy coat samples obtained from the Madrid Blood Center under a research agreement. PBMC were cultured at 37°C in supplemented RPMI media with 1% mix of antibiotics (125 µg/mL ampicillin, 125 µg/mL cloxacillin, and 40 µg/mL gentamicin) and 5% FBS.

Primary ThyTreg cells and lentiviral transduction: All experiments in primary cells were performed on ThyTreg isolated from de-identified thymus obtained from pediatric cardiac surgery (infants without syndrome affecting the thymus (such as Down syndrome or DiGeorge syndrome) as described in PCT/EP2019/055221, Example 1. ThyTreg were cultured at 37°C in X-vivo15 supplemented with 5% human serum (Sigma-Aldrich) instead of FBS. And the media was supplemented with 600 U/ml human recombinant IL-2 (Immunotools). Briefly, thymus was then disaggregated mechanically (gentleMACS^{™} Dissociators) and ThyTreg from thymocytes were selected using CD25 MicroBeads II (human, miltenyi Biotec). ThyTreg were stimulated and expanded using antiCD3/CD28 beads (Dynabeads). For transduction, 24 hours after stimulation, lentivirus, as obtained above, was added to cells at an MOI of 1-50. After 48 hours, cells were washed and resuspended in fresh ThyTreg media containing 600 U/ml IL-2. After an additional 2 days of stimulation and expansion, the resulting cells were flow-sorted by EGFP expression. Sorted CAR-ThyTreg then underwent additional 2 days of culture prior to being assayed.

Flow cytometry staining: ThyTreg and CAR-ThyTreg (CARCD28 or CAR41-BB) were stained using the indicated antibodies in staining buffer (PBS + 2% FBS), for 30 min at 4°C. Alternatively, CAR expression on ThyTreg cells was determined by using the biomolecules Atto 665 Biotin in PBS (Merck Sigma-Aldrich), for 60 min at room temperature. After two washes with PBS, cells were stained with Fixable lity Dye (Miltenyi Biotec) 30 min at 4°C in PBS and washed twice with staining buffer. Live cells were gated based on forward (FSC) and the signal negative for Fixable Viability Dye (Fig. 6A). CAR-ThyTreg were gated on double positive cells EGFP and Atto 665 Biotin (Fig. 6B). 50,000 total events were recorded per sample. The flow cytometry parameters are adjusted using isotypic controls and utilizing fluorescence minus one (FMO) methodology.

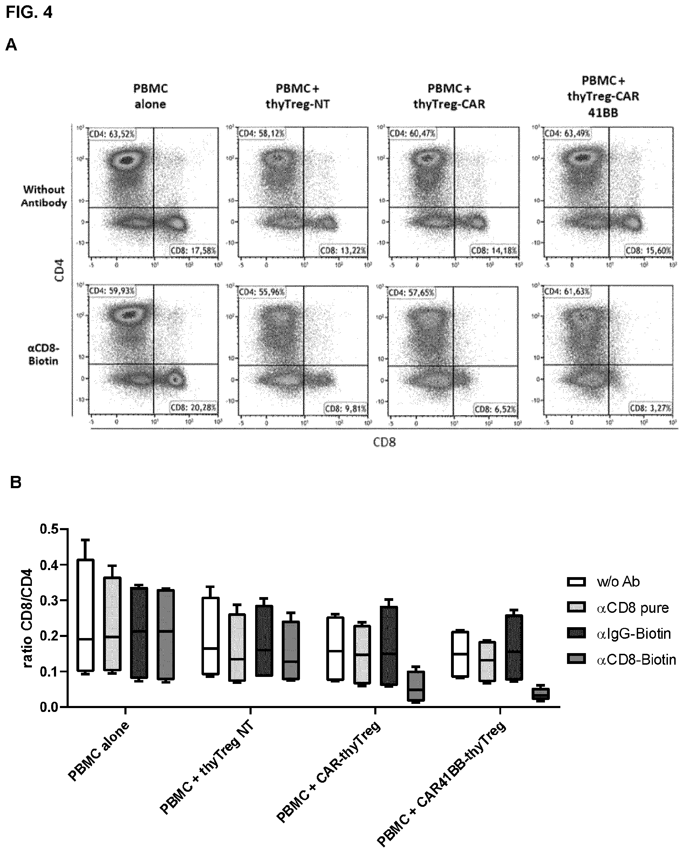

Suppressive function assay (Fig. 4-5-7): the co-culture experiments were based on a series of controls, target cells cultured alone, cultured with ThyTreg or CAR-ThyTreg with or without biotinylated antibody. Moreover, a specific biotinylated antibody and several other antibodies were used as negative controls: the specific non-biotinylated antibody and an unspecific biotinylated antibody. The target cells were PBMC. CD8 T cells from PBMC were considered the real target cells since the biotinylated antibody was anti-CD8. The non-CD8 T cells were used as off-target controls. The day before co-cultures, target PBMCs were stained with CELLTRACE^{™} Violet (ThermoFisher) following the manufacturer's instructions. 1 million were then activated with 1 million antiCD3/CD28 magnetic beads (Dynabeads^{™}) for 18 hours in a 24-well plate, in 2 ml of X-vivo15 complemented with 5% Human Serum and 60 U/ml IL-2. 1 million effector cells ThyTreg or CAR-ThyTreg) were then activated with 1 million antiCD3/CD28 magnetic beads (Dynabeads^{™}) for 18 hours in a 24-well plate, in 2 ml of X-vivo15 complemented with 5% Human Serum and 600 U/ml IL-2. After 18 hours of stimulation, beads were removed, and effector and target cells were washed with pre-warm X-vivo15 complemented with 5% Human Serum and 600 U/ml IL-2. 50,000 CellTrace-violet-PBMC were cultured alone or incubated for 24 or 72 hours with 100,000 ThyTreg or CAR-ThyTreg in 250 µl in a 96 well-U-bottom plate (ratio ThyTreg: target ratio of 2:1) in X-vivo15 complemented with 5% Human Serum and 600 U/ml IL-2 without antibodies or with 1,5 µg/ml of anti-CD8-biotin, anti-IgG-biotin or anti-CD8 pure. Cells alone and co-cultured cells were incubated 24 or 72 hours at 37°C in X-vivo15 complemented with 5% Human Serum and 600 U/ml IL-2. After 72 hours post-coculture, cells were harvested, and effector cells (ThyTreg or CAR-ThyTreg) were differentiated from target PBMC since PBMC were labelled CELLTRACE violet by flow cytometry. Harvested cells were labelled in different cytometry panels for superficial markers: CD8-APC/Cy5 (Beckman Coulter), and CD4-ECD (Miltenyi Biotec). The target cell proliferation was followed by the loss of the CELLTRACE violet signal using flow cytometry analysis. The viability of target cells was monitored using 7-Aminoactinomycin D (7-AAD), which produces a positive signal in cells undergoing death, as measured by flow cytometry.

The percentage of suppression was calculated by comparing the percentage of target cell proliferation co-cultured with the effector cells against the percentage of target cell proliferation cultured alone.

### Results

Lentivectors encoding the two mSA2-CARs with two intracellular signalling domains (FIG. 1 and FIG. 2), mSA2-CD28-CD3ζ (also referred as "CARCD28", comparative purpose) and mSA2-41BB-CD3ζ (also referred as "CAR41-BB", embodiment of the invention) were constructed.

Driven by the EF1alpha promoter, the CAR-coding region is formed by the murine igk leader sequence, the codon-optimized mSA2, the CD8alpha hinge domain, the CD28 transmembrane domain, either 41BB or CD28 signaling domain and the CD3ζ cytoplasmic domain. Additionally, the EGFP gene was added via a T2A co-translation peptide. All the vectors were packaged into lentivirus and transduced into primary human ThyTreg cells.

After three days of stimulation and two days of transduction, transduced ThyTreg were evaluated for their viability (FIG 6A, analysed with 7AAD) and for the frequency of transduction using ATTO665-biotin labeling and GFP expression followed by flow cytometry (Fig 6B). It was found that the staining with ATTO665-biotin was specific for CAR expression since ATTO665-biotin and EGFP expression correlated. Therefore, by flow cytometry, mSA2-CAR was found to be expressed on the ThyTreg cell surface. Then, for the cells that expressed less than 30% of CAR, cells were sorted.

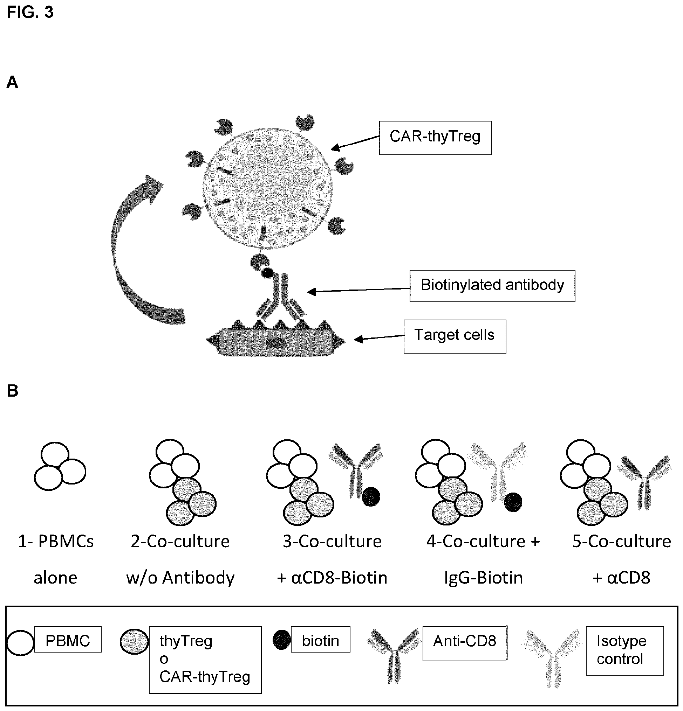

Next, it was tested if CAR-ThyTreg could be combined with a specific antibody to mediate specific ThyTreg functions. For these studies, ThyTreg (isolated from thymus tissue), CARCD28 and CAR41-BB ThyTreg cells were co-cultured with primary allogeneic human PBMC (FIG. 3) without (w/o) antibody, with a non-biotinylated specific antibody (anti-CD8 pure), with biotinylated non-specific antibody (anti-IgG-Biotin) or biotinylated specific antibody (anti-CD8-Biotin). Following three days of co-cultures of effectors cells (ThyTreg) and target cells (PBMC-CELLTRACE violet), the cells were harvested and labelled for determining viability, CD4 and CD8 subsets in the target cells determined as CELLTRACE violet positive cells.

It was found that the CD8 population disappeared when co-culture with engineered CAR41-BB ThyTreg (FIG. 4A). Interestingly, analyzing all the culture conditions, it was also found that the ratio of CD8:CD4 in the target cells was modified only when the co-culture was PBMC + CAR4-1 BB-ThyTreg + anti-CD8-Biotin conditions, with an evident diminution of CD8 subset frequency compared to CD4 subset. Moreover, this diminution in the ratio was not detected when the co-culture was done with ThyTreg non-treated or when PBMC were cultured alone with antibodies (FIG. 4B). Therefore, and importantly, the presence of the specific biotinylated antibody and target cells (in this case, CD8) was required for all the genetically modified ThyTreg to be activated.

Cell death is one mechanism used to suppress effector cells. Therefore, in this co-culture experiment (FIG 4), we investigated whether the disappearance of CD8 was due to the induction of cell death. Analyzing the frequencies of 7AAD+ cells, we showed that the suppressive function induced by the genetically modified ThyTreg compared to the non-modified ThyTreg (ThyTreg-NT) was due to specific cell death (Fig 5). Interestingly, analyzing all the culture conditions, it was found that the lowest viability of the CD8 subset into the total PBMC target cells was achieved when the co-culture was PBMC + CAR4-1 BB-ThyTreg + anti-CD8-Biotin conditions (FIG. 5A), with a clear low CD8 subset viability compared to the maintain viability of the CD4 subset (FIG. 5B). Moreover, this diminution in the frequency was not detected when the co-culture was done with non-treated ThyTreg or when PBMC were cultured alone with antibodies. The specific biotinylated antibody and target cells (in this case, CD8) were required for all the genetically modified ThyTreg to be activated and functional. It is important to note that cell death was not induced by polyclonal ThyTreg. This point makes a novel difference in the function of the CAR-ThyTreg compared with the initial polyclonal and non-modified ThyTreg product.

On the other hand, the suppressive function may be induced by a suppression of the cellular proliferation. We observed that the genetically modified thyTreg compared to the non-modified ThyTreg (ThyTreg-NT) were able to induce a suppression of proliferation. Indeed, the disappearance of the CD8 target subset observed in FIG. 4, compared to the CD4 subset, was also due to a suppression of cell proliferation. Analyzing all the culture conditions, it was found that the proliferation ability of the CD8 subset into the total PBMC target cells was diminished when the co-culture was PBMC + CAR4-1BB-ThyTreg + anti-CD8-Biotin conditions (FIG. 7, light grey) compared to the suppression of the cellular proliferation of the CD4 subset (dark grey). Moreover, the PBMC + CAR4-1 BB-ThyTreg + anti-CD8-Biotin condition show the higher suppressive ability (82% of suppression on CD8+ T cells (+/- SEM: 7.1%) and 53.7% of suppression for CD4+ T cells (+/- SEM 18.8%)) and killing ability (FIG 5). This suppression was higher than that observed for the ThyTreg NT with 47.9% of suppression on CD8+T cells (+/- SEM 6.2%) and 30.6% of suppression on CD4+T cells (+/- SEM 22.1%).

The cells CAR-ThyTreg based on mSA2 has a high potential specific suppressive capacity that improves those found for polyclonal ThyTreg. Besides, the CAR-ThyTreg of the invention were found to suppress target cells through a different mechanism than polyclonal ThyTreg, inducing target cell death. These cells can be used with the antibodies already approved by the FDA or DEA or currently in clinical development. Therefore, the cells can be used as an off-the-shelf reagent for preselecting in vitro the best candidate antibodies for antigen-binding domains of traditional CAR before proceeding with their construction.

### BIBLIOGRAPHIC REFERENCES

Beheshti, S.A., Shamsasenjan, K., Ahmadi, M., Abbasi, B., 2022. CAR Treg: A new approach in the treatment of autoimmune diseases. Int Immunopharmacol 102, 108409.
Bluestone, J.A., Buckner, J.H., Fitch, M., Gitelman, S.E., Gupta, S., Hellerstein, M.K., Herold, K.C., Lares, A., Lee, M.R., Li, K., Liu, W., Long, S.A., Masiello, L.M., Nguyen, V.,
Putnam, A.L., Rieck, M., Sayre, P.H., Tang, Q., 2015. Type 1 diabetes immunotherapy using polyclonal regulatory T cells. Sci Transl Med 7, 315ra189.
Brunstein, C.G., Miller, J.S., McKenna, D.H., Hippen, K.L., DeFor, T.E., Sumstad, D., Curtsinger, J., Verneris, M.R., MacMillan, M.L., Levine, B.L., Riley, J.L., June, C.H., Le, C., Weisdorf, D.J., McGlave, P.B., Blazar, B.R., Wagner, J.E., 2016. Umbilical cord blood-derived T regulatory cells to prevent GVHD: kinetics, toxicity profile, and clinical effect. Blood 127, 1044-1051.
Chwojnicki, K., Iwaszkiewicz-Grze 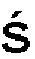 , D., Jankowska, A., Zieli 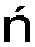 ski, M., 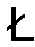 owiec, P., Gliwi 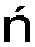 ski, M., Grzywi 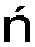 ska, M., Kowalczyk, K., Konarzewska, A., Glasner, P., Sakowska, J., Kulczycka, J., Ja 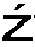 wi 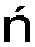 ska-Cury 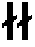 o, A., Kubach, M., Karaszewski, B., Nyka, W., Szurowska, E., Trzonkowski, P., 2021. Administration of CD4. BioDrugs 35, 47-60.
Dawson, N.A.J., Rosado-S 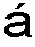 nchez, I., Novakovsky, G.E., Fung, V.C.W., Huang, Q., McIver, E., Sun, G., Gillies, J., Speck, M., Orban, P.C., Mojibian, M., Levings, M.K., 2020. Functional effects of chimeric antigen receptor co-receptor signaling domains in human regulatory T cells. Sci Transl Med 12.
Gliwi 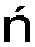 ski, M., Iwaszkiewicz-Grze 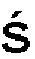 , D., Wo 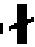 oszyn-Durkiewicz, A., Tarnowska, M., Żali 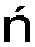 ska, M., Hennig, M., Zieli 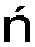 ska, H., Dukat-Mazurek, A., Zielkowska-D 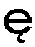 bska, J., Zieli 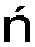 ski, M., Ja 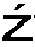 wi 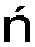 ska-Cury 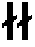 o, A., Owczuk, R., Jarosz-Chobot, P., Bossowski, A., Szadkowska, A., M 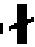 ynarski, W., Marek-Trzonkowska, N., Moszkowska, G., Siebert, J., My 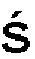 liwiec, M., Trzonkowski, P., 2020. Proinsulin-specific T regulatory cells may control immune responses in type 1 diabetes: implications for adoptive therapy. BMJ Open Diabetes Res Care 8.
Juneja, T., Kazmi, M., Mellace, M., Saidi, R.F., 2022. Utilization of Treg Cells in Solid Organ Transplantation. Front Immunol 13, 746889.
Ma, Y., He, K.M., Garcia, B., Min, W., Jevnikar, A., Zhang, Z.X., 2008. Adoptive transfer of double negative T regulatory cells induces B-cell death in vivo and alters rejection pattern of rat-to-mouse heart transplantation. Xenotransplantation 15, 56-63.
Marek-Trzonkowska, N., My 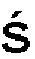 liwiec, M., Dobyszuk, A., Grabowska, M., Derkowska, I., Ju 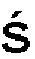 ci 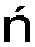 ska, J., Owczuk, R., Szadkowska, A., Witkowski, P., M 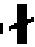 ynarski, W., Jarosz-Chobot, P., Bossowski, A., Siebert, J., Trzonkowski, P., 2014. Therapy of type 1 diabetes with CD4(+)CD25(high)CD127-regulatory T cells prolongs survival of pancreatic islets - results of one year follow-up. Clin Immunol 153, 23-30.
Safinia, N., Vaikunthanathan, T., Fraser, H., Thirkell, S., Lowe, K., Blackmore, L., Whitehouse, G., Martinez-Llordella, M., Jassem, W., Sanchez-Fueyo, A., Lechler, R.I., Lombardi, G., 2016. Successful expansion of functional and stable regulatory T cells for immunotherapy in liver transplantation. Oncotarget 7, 7563-7577.
Lohmueller, J.J., et al., mSA2 affinity-enhanced biotin-binding CAR T cells for universal tumor targeting. Oncoimmunology, 2017. 7(1): p. e1368604.
Sagoo, P., Ali, N., Garg, G., Nestle, F.O., Lechler, R.I., Lombardi, G., 2011. Human regulatory T cells with alloantigen specificity are more potent inhibitors of alloimmune skin graft damage than polyclonal regulatory T cells. Sci Transl Med 3, 83ra42.
Sakaguchi, S., Yamaguchi, T., Nomura, T., Ono, M., 2008. Regulatory T cells and immune tolerance. Cell 133, 775-787. Salmon, P., Trono, D., 2006. Production and titration of lentiviral vectors. Curr Protoc Neurosci Chapter 4, Unit 4.21.
Sicard, A., Koenig, A., Morelon, E., Defrance, T., Thaunat, O., 2015. Cell therapy to induce allograft tolerance: time to switch to plan B? Front Immunol 6, 149. Trzonkowski, P., Bieniaszewska, M., Ju 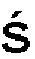 ci 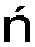 ska, J., Dobyszuk, A., Krzystyniak, A., Marek, N., My 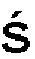 liwska, J., Hellmann, A., 2009. First-in-man clinical results of the treatment of patients with graft versus host disease with human ex vivo expanded CD4+CD25+CD127- T regulatory cells. Clin Immunol 133, 22-26.

### CLAUSES

For reasons of completeness, various aspects of the invention are set out in the following numbered clauses:
**Clause 1.** A thymus T regulation cell (ThyTreg cell) which codes for, or alternatively expresses on its surface, a chimeric antigen receptor (CAR) comprising an extracellular domain, a hinge region, a transmembrane domain and an intracellular domain, wherein the intracellular domain comprises:
   a cytoplasmatic co-stimulation domain with a sequence having an identity of at least 85% with respect to SEQ ID NO: 1; and
   a cytoplasmatic stimulation domain.
**Clause 2.** The ThyTreg cell of clause 1, wherein the extracellular domain comprises or consists of a biotin-binding moiety; particularly the biotin-binding moiety is selected from: neutravidin, bradavidin, tamavidin, shwanavidin, zebavidin, streptavidin, streptavidin derivatives, or any functional fragment thereof; particularly the biotin-binding moiety is streptavidin or a functional fragment thereof; particularly the biotin-binding moiety is monomeric streptavidin (mSA2).
**Clause 3.** The ThyTreg cell of any one of the clauses 1-2, wherein the cytoplasmatic co-stimulation domain consists of SEQ ID NO: 1.
**Clause 4.** The ThyTreg cell of any one of the preceding clauses, wherein the cytoplasmatic stimulation domain is CD3zeta.
**Clause 5.** The ThyTreg cell of any one of the preceding clauses, wherein the transmembrane domain is selected from CD28, CD3, CD45, CD4, CD8, CD9, CD16, CD22, CD33, CD37, CD64, CD80, CD86, CD134, CD137, or CD154; particularly is CD28 (SEQ ID NO: 4).
**Clause 6.** The ThyTreg cell of any one of the preceding clauses, wherein the hinge region originates from CD8a (SEQ ID NO: 3).
**Clause 7.** The ThyTreg cell of any one of the preceding clauses, wherein the CAR comprises (i) mSA2, (ii) CD8a hinge region, (iii) CD28 transmembrane region, (iv) SEQ ID NO: 1, and (v) CD3zeta, wherein (i) to (v) are provided in N-terminal to C-terminal order; particularly it has at least 85% of identity with respect to sequences SEQ ID NO: 6.
**Clause 8.** The ThyTreg cell of any one of the preceding clauses, wherein the extracellular domain does not include neither is fused to an antibody or fragment thereof.
**Clause 9.** The ThyTreg cell of any one of the preceding clauses 1-7, wherein the extracellular domain is fused to a binding moiety specific of an activated immune cell, particularly an antibody or fragment thereof, more particularly an anti-CD8+ antibody or fragment thereof.
**Clause 10.** A method for preparing a ThyTreg cell as defined in any one of the preceding clauses 1-9 which comprises the step of transfecting isolated ThyTreg cells with an expression vector codifying for the CAR as defined in any of the preceding clauses.
**Clause 11.** The method of clause 10, wherein the expression vector is a virus, particularly a lentivirus.
**Clause 12.** The method of any one of the clauses 10-11, wherein the expression vector comprises a sequence with at least 85% of identity with SEQ ID NO: 7; particularly, the expression vector comprises a sequence 100% identical to SEQ ID NO: 7.
**Clause 13.** The method of any one of the clauses 10-12, wherein the expression vector has sequence with at least 85% of identity with respect to sequence SEQ ID NO: 8; particularly, the expression vector is 100% identical to SEQ ID NO: 8.
**Clause 14.** The method of any one of the clauses 10-13, wherein the thymic Treg cells are obtained following the steps:
   a. mechanically disaggregating an isolated thymic tissue;
   b. filtering the product obtained after stage (a), and resuspending the precipitate comprising thymocytes in a culture medium;
   c. isolating CD25+ cells from the product obtained after stage (b);
   d. culturing the cell population obtained after stage (c) in a culture medium in the presence of a T cell activator and IL-2, wherein said T cell activator comprises at least CD3 and CD28 agonists; and
   e. removing the T cell activator from the culture medium of stage (d);
   the method optionally further comprising a step (f) culturing the regulatory T cells in a culture medium in the presence of IL-2; with the proviso that prior to step (d) the cell population has not been depleted from CD8+ cells.
**Clause 15.** The method according to clause 14, wherein step (a) comprises mechanically disaggregating the thymic tissue in the presence of a culture medium and without using enzymes.
**Clause 16.** The method according to anyone of clauses 14-15, wherein isolation of CD25+ cells in step (c) comprises the use of magnetic beads conjugated to antibodies against CD25.
**Clause 17.** The method according to anyone of clauses 14-16, wherein the T cell activator of stage (d) is a colloidal polymer nanomatrix conjugated with humanised CD3 and CD28 agonists.
**Clause 18.** The method according to anyone of clauses 14-17, wherein the T cell activator of stage (d) is used in a T cell activator: cell ratio range between 1:10 and 1:100.
**Clause 19.** The method according to anyone of clauses 14-18, wherein the cells are cultured in step (d) for at least 2 or 3 days, preferably for at least 3 days.
**Clause 20.** The method, according to anyone of clauses 14-19, wherein the cells are cultured in step (d) in the absence of rapamycin.
**Clause 21.** The method according to anyone of clauses 14-20, wherein the removal of stage (e) is carried out by a magnet or centrifugation.
**Clause 22.** The method according to anyone of clauses 14-21, wherein in step (f) the regulatory T cells are cultured for another one to seven days, preferably for another four days in the presence of IL-2.
**Clause 23.** The method, according to anyone of clauses 14-22, wherein the cells are cultured in step (f) in the absence of rapamycin.
**Clause 24.** The method according to anyone of clauses 14-23, wherein said culture medium is a GMP culture medium.
**Clause 25.** The method according to anyone of clauses 14-24, wherein said culture medium further comprises antibiotic, preferably 5% of antibiotic.
**Clause 26.** The method, according to anyone of clauses 14-25, wherein the thymic tissue comes from a human.
**Clause 27.** The ThyTreg cell obtainable by the method of any one of the clauses 10-26.
**Clause 28.** A pharmaceutical composition comprising a therapeutically effective amount of the CAR-ThyTreg cells as defined in anyone of the clauses 1 to 8 or 27, and one or more pharmaceutically acceptable excipients or carriers.
**Clause 29.** A combination comprising
   a Treg cell population of ThyTreg cells as defined in any of the preceding clauses 2-8, and
   a binding moiety specific of an activated immune cell, this moiety being conjugated to biotin.
**Clause 30.** The combination of clause 29, wherein the binding moiety specific of an activated cell is an antibody or fragment thereof.
**Clause 31.** The combination of clause 30, wherein the antibody is anti-CD8+.
**Clause 32.** The combination of any one of the clauses 29-31, wherein the ThyTreg cells has an extracellular domain consisting of mSA2.
**Clause 33.** A chimeric antigen receptor (CAR) comprising an extracellular domain, a hinge region, a transmembrane domain and an intracellular domain, wherein
   the extracellular domain comprises an antibody anti-CD8+ or a fragment thereof; and the intracellular domain comprises:
   a cytoplasmatic co-stimulation domain with a sequence having an identity of at least 85% with respect to SEQ ID NO: 1; and
   a cytoplasmatic stimulation domain.
**Clause 34.** A nucleic acid encoding the CAR as defined in clause 34.
**Clause 35.** An expression vector comprising the nucleic acid of clause 35.
**Clause 36.** A cell transduced with the vector of clause 36.
**Clause 37.** The cell of clause 37, which is a Treg cell, particularly a thyTreg cell.
**Clause 38.** A pharmaceutical composition comprising an effective amount of the vector of clause 36 or a therapeutically effective amount of the cells transduced with the vector of clause 36, and one or more pharmaceutically acceptable vehicles or carriers.
**Clause 39.** The CAR-ThyTreg cell as defined in anyone of the clauses 1-8, 27 or 36, the pharmaceutical composition of clause 28 or 38, or the combination of any one of the clauses 29-32, for use in therapy or diagnosis.
**Clause 40.** A CAR-ThyTreg cell as defined in anyone of the clauses 1-8, 27 or 36-37, the pharmaceutical composition of clause 28 or 39, or the combination of any one of the clauses 29-32 for use in a method for inducing or restoring tolerance to the immune system.
**Clause 41.** A CAR-ThyTreg cell as defined in anyone of the clauses 1-8, 27 or 36-37, the pharmaceutical composition of clause 28 or 39, or the combination of any one of the clauses 29-32 for use in a method of treatment of a disease selected from: autoimmune disease, inflammatory processes, allergy, graft-versus host disease and immune rejection to transplant.
**Clause 42.** The combination of any one of clauses 29-31 for use according to any of the clauses 40 to 41, wherein the method comprises the separate administration of the ThyTreg cell population and binding moiety specific of an activated immune cell, to the subject.

## Claims

1. A thymus T regulation cell (ThyTreg cell) which codes for, or alternatively expresses on its surface, a chimeric antigen receptor (CAR) comprising an extracellular domain, a hinge region, a transmembrane domain and an intracellular domain, wherein the intracellular domain comprises:
a cytoplasmatic co-stimulation domain with a sequence having an identity of at least 85% with respect to SEQ ID NO: 1; and
a cytoplasmatic stimulation domain.

2. The ThyTreg cell of claim 1, wherein the extracellular domain comprises or consists of a biotin-binding moiety; particularly the biotin-binding moiety is selected from: neutravidin, bradavidin, tamavidin, shwanavidin, zebavidin, streptavidin, streptavidin derivatives, or any functional fragment thereof; particularly the biotin-binding moiety is streptavidin or a functional fragment thereof; particularly the biotin-binding moiety is monomeric streptavidin (mSA2).

3. The ThyTreg cell of any one of the claims 1-2, wherein the cytoplasmatic co-stimulation domain consists of SEQ ID NO: 1.

4. The ThyTreg cell of any one of the preceding claims, wherein the cytoplasmatic stimulation domain is CD3zeta.

5. The ThyTreg cell of any one of the preceding claims, wherein the transmembrane domain is selected from CD28, CD3, CD45, CD4, CD8, CD9, CD16, CD22, CD33, CD37, CD64, CD80, CD86, CD134, CD137, or CD154; particularly is CD28 (SEQ ID NO: 4).

6. The ThyTreg cell of any one of the preceding claims, wherein the hinge region originates from CD8a (SEQ ID NO: 3).

7. The ThyTreg cell of any one of the preceding claims, wherein the CAR comprises (i) mSA2, (ii) CD8a hinge region, (iii) CD28 transmembrane region, (iv) SEQ ID NO: 1, and (v) CD3zeta, wherein (i) to (v) are provided in N-terminal to C-terminal order; particularly it has at least 85% of identity with respect to sequences SEQ ID NO: 6.

8. A method for preparing a ThyTreg cell as defined in any one of the preceding claims 1-7 which comprises the step of transfecting isolated ThyTreg cells with an expression vector codifying for the CAR as defined in any of the preceding claims.

9. The method of claim 8, wherein the expression vector:
comprises or consists of a sequence with at least 85% of identity with SEQ ID NO: 7; particularly, the expression vector comprises a sequence 100% identical to SEQ ID NO: 7; or
has sequence with at least 85% of identity with respect to sequence SEQ ID NO: 8; particularly, the expression vector is 100% identical to SEQ ID NO: 8.

10. The method of any one of the claims 8-9, wherein the isolated ThyTreg cells are obtained following the steps:
a. mechanically disaggregating an isolated thymic tissue;
b. filtering the product obtained after stage (a), and resuspending the precipitate comprising thymocytes in a culture medium;
c. isolating CD25+ cells from the product obtained after stage (b);
d. culturing the cell population obtained after stage (c) in a culture medium in the presence of a T cell activator and IL-2, wherein said T cell activator comprises at least CD3 and CD28 agonists; and
e. removing the T cell activator from the culture medium of stage (d);
the method optionally further comprising a step (f) culturing the regulatory T cells in a culture medium in the presence of IL-2; with the proviso that prior to step (d) the cell population has not been depleted from CD8+ cells.

11. A pharmaceutical composition comprising a therapeutically effective amount of the CAR-ThyTreg cells as defined in anyone of the claims 1 to 7, and one or more pharmaceutically acceptable excipients or carriers.

12. A combination comprising
a Treg cell population of ThyTreg cells as defined in any of the preceding claims 2-7, and
a binding moiety specific of an activated immune cell, particularly an antibody or fragment thereof, this moiety being conjugated to biotin.

13. The CAR-ThyTreg cell as defined in anyone of the claims 1-7, the pharmaceutical composition of claim 11 or the combination of claim 12, for use in therapy or diagnosis.

14. A CAR-ThyTreg cell as defined in anyone of the claims 1-7, the pharmaceutical composition of claim 11 or the combination of claim 12, for use in immunotherapy, particularly in a method for inducing or restoring tolerance to the immune system; particularly in a method for the treatment of a disease selected from: autoimmune disease, inflammatory processes, allergy, graft-versus host disease and immune rejection to transplant.

15. The combination of 12 for use according to claim 14, wherein the method comprises the separate administration of the ThyTreg cell population and binding moiety specific of an activated immune cell, to the subject.
